(19) **Europäisches Patentamt**
**European Patent Office**
**Office européen des brevets**

(11) **EP 4 656 738 A1**

(12) **EUROPEAN PATENT APPLICATION**
published in accordance with Art. 153(4) EPC

(43) Date of publication:
**03.12.2025 Bulletin 2025/49**

(21) Application number: **24747438.0**

(22) Date of filing: **24.01.2024**

(51) International Patent Classification (IPC):
**C12Q 1/6886** (2018.01)

(52) Cooperative Patent Classification (CPC):
**C12Q 1/6886**

(86) International application number:
**PCT/KR2024/001115**

(87) International publication number:
**WO 2024/158203 (02.08.2024 Gazette 2024/31)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.01.2023 KR 20230010403**

(71) Applicant: **Lepidyne Co., Ltd.**
**Seongdong-gu**
**Seoul**
**04779 (KR)**

(72) Inventors:
• **KIM, Young-Joon**
**Seoul 06024 (KR)**

• **KIM, Si-Cho**
**Seoul 06024 (KR)**
• **HA, Jeong-Sil**
**Seoul 06024 (KR)**
• **KIM, Da Won**
**Seoul 06366 (KR)**

(74) Representative: **Grünecker Patent- und
Rechtsanwälte
PartG mbB
Leopoldstraße 4
80802 München (DE)**

Remarks:
The complete document including Reference
Table(s) and the Sequence Listing(s) can be
downloaded from the EPO website

(54) **COMPOSITION FOR PREDICTING RISK OF DEVELOPING LIVER CANCER**

(57) The present application relates to a composition for predicting a risk of developing liver cancer.

【FIG. 9b】

EP 4 656 738 A1

**Description**

[TECHNICAL FIELD]

The present application relates to a composition for predicting a risk of developing liver cancer.

[BACKGROUND ART]

Although numerous cells in the human body have the same DNA base sequence, the form and function of each cell are present very variously. This is because a specific gene is expressed in each cell, and according to this, each cell retains an independent phenotype through different differentiation processes from each other. Various factors such as DNA methylation, histone modification, tissue specific transcription factors and the like are involved in gene expression which is different in each cell.

Liquid biopsy is a method for examining by securing samples through non-invasion or minimal invasion, and it is a method that places less burden on a test subject than conventionally used tissue biopsy, enables continuous monitoring through frequent checkup, and there is no risk of radiation exposure which is a side effect of radiological methods.

The survival rate of cancer patients shows significant differences depending on the clinical stage at the time of diagnosis, and treatment based on early detection of cancer can ensure a high survival rate of patients. In case of liver cancer, it is currently showing an increasing incidence rate worldwide, and most patients are diagnosed at a late clinical stage of cancer. Therefore, development of a method for diagnosing liver cancer at an early stage is considered a technology which can bring about social and economic benefits.

[DISCLOSURE]

[TECHNICAL PROBLEM]

One embodiment of the present application is to provide a composition for predicting a risk of developing liver cancer, or a method for providing information for predicting a risk of developing liver cancer, which can predict a risk of developing liver cancer using a biomarker specific to liver cancer, and detect the marker in blood-derived cell-free DNA (cfDNA).

[TECHNICAL SOLUTION]

One embodiment of the present application relates to a composition for predicting a risk of developing liver cancer, comprising an agent capable of measuring a methylation level of at least one of CpG sites comprised in the 21810279th to 21810792nd sequence region of the human chromosome 12, the 29298021st to 29298631st sequence region of the human chromosome 17, or the 19738547th to 19739846th sequence region of the human chromosome 19, in a biological sample.

Another embodiment of the present invention relates to a method for providing information for predicting a risk of developing liver cancer, or a method for predicting a risk of developing liver cancer, comprising a step of measuring a methylation level of at least one of CpG sites comprised in the 21810279th to 21810792nd sequence region of the human chromosome 12, the 29298021st to 29298631st sequence region of the human chromosome 17, or the 19738547th to 19739846th sequence region of the human chromosome 19, in a biological sample isolated from a subject.

Hereinafter, the present application will be described in more detail.

In the present description, the term "methylation" means that a methyl group is attached to a base composing DNA. For example, methylation may occur in cytosine of a specific CpG site of a specific gene or nucleic acid.

In the present description, the term "whether methylated" or "methylation status" means whether methylation occurs on cytosine of a specific CpG site of a specific gene or nucleic acid or not. Specifically, it refers to presence or absence of 5-methyl-cytosine of at least one CpG dinucleotide in a base sequence. For example, it may be whether methylation occurs or not on cytosine of at least one CpG site comprised in the sequence represented by SEQ ID NO: 1, sequence represented by SEQ ID NO: 2, or sequence represented by SEQ ID NO: 3.

In the present description, the term "methylation level" or "methylation degree" means an amount of methylation present in a base sequence in the sequence represented by SEQ ID NO: 1, sequence represented by SEQ ID NO: 2, or sequence represented by SEQ ID NO: 3.

In the present description, the term, "CpG site" or "CpG sequence" means a CpG site present on a base sequence of a specific gene or nucleic acid. The gene is a concept including all of a series of constituent units which are needed for expression and are operably linked to each other, and for example, may comprise a promoter region, a protein encoding region (open reading frame, ORF) and a terminator region. Therefore, the CpG site may be present in the promoter region, protein encoding region (open reading frame, ORF), or terminator region or the like of the corresponding gene. For example, it may be a CpG site present in the promoter region of the gene.

In the present description, the term, "nucleic acid" is a nucleotide in a polymer form, and represents a ribonucleotide or deoxyribonucleotide, and includes meanings of a polynucleotide, an oligonucleotide, an oligomer, an oligo, a coding sequence, and the like. The nucleic acid may be used as a meaning including polymers having single-, double- or multi-stranded DNA or RNA, genomic DNA, cDNA, a DNA-RNA hybrid, or purine and pyrimidine bases, or other natural, chemically or biochemically modified, non-natural or derivatized nucleotide base. For example, the nucleic acid comprises at least one CpG site comprised in the nucleic acid represented by SEQ ID NO: 1, SEQ ID NO: 2, or SEQ ID NO: 3, and it may be a nucleic acid having a continuous partial sequence of the nucleic acid represented by SEQ ID NO: 1, SEQ ID NO: 2, or SEQ ID NO: 3. As one example, the nucleic acid may be the nucleic acid represented by SEQ ID NO: 1, SEQ ID NO: 2, or SEQ ID NO: 3.

In the present description, when it is described that a component is "comprised", this means that other components may be further comprised rather than excluding other components, unless specifically described to the contrary.

In the present description, unless otherwise indicated, a nucleic acid is described from left to right, in the 5' to 3' direction, respectively.

In the present description, a singular form includes plural objects unless the context clearly indicates otherwise.

One embodiment of the present application relates to a composition for predicting a risk of developing liver cancer, comprising an agent capable of measuring a methylation level of at least one CpG site comprised in the 21810279th to 21810792nd sequence region of the human chromosome 12, the 29298021st to 29298631st sequence region of the human chromosome 17, or the 19738547th to 19739846th sequence region of the human chromosome 19 in a biological sample.

Specifically, the composition according to one embodiment of the present application may comprise an agent capable of measuring a methylation level of at least one CpG site comprised in the 21810279th to 21810792nd sequence region of the human chromosome 12 in a biological sample, and further comprise an agent capable of measuring a methylation level of at least one CpG site comprised in the 29298021st to 29298631st sequence region of the human chromosome 17; and/or an agent capable of measuring a methylation level of at least one CpG site comprised in the 19738547th to 19739846th sequence region of the human chromosome 19. According to the Example of the present application, as a result of measuring all of the methylation level of at least one CpG site comprised in the 21810279th to 21810792nd sequence region of the human chromosome 12, and the methylation level of at least one CpG site comprised in the 29298021st to 29298631st sequence region of the human chromosome 17, and/or the methylation level of at least one CpG site comprised in the 19738547th to 19739846th sequence region of the human chromosome 19, tumor coverage is further improved, and therefore, the accuracy of prediction of a risk of liver cancer increased.

The composition may further comprise an agent capable of measuring the concentration of alpha fetoprotein (AFP) in the biological sample.

The sequence region may be comprised in cell free DNA (cfDNA).

"Biomarker" or "marker" according to one embodiment of the present application, a general term for nucleic acids comprising at least one CpG site comprised in the 21810279th to 21810792nd sequence region of the human chromosome 12, the 29298021st to 29298631st sequence region of the human chromosome 17, or the 19738547th to 19739846th sequence region of the human chromosome 19.

At least one CpG site comprised in the 21810279th to 21810792nd sequence region of the human chromosome 12 may be at least one selected from the group consisting of a CpG site positioned in the 21810279th to 21810280th sequence region of the human chromosome 12, a CpG site positioned in the 21810450th to 21810451st sequences, a CpG site positioned in the 21810458th to 21810459th sequences, a CpG site positioned in the 21810489th to 21810490th sequences, a CpG site positioned in the 21810600th to 21810601st sequences, a CpG site positioned in the 21810750th to 21810751st sequences, a CpG site positioned in the 21810759th to 21810760th sequences, and a CpG site positioned in the 21810791st to 21810792nd sequence, and as one example, it may comprise a CpG site positioned in the 21810750th to 21810751st sequence region of the human chromosome 12. The 21810279th to 21810792nd sequence region of the human chromosome 12 may have the sequence represented by SEQ ID NO: 1. In the present description, the nucleic acid comprising at least one CpG site comprised in the 21810279th to 21810792nd sequence region of the human chromosome 12 is named "Marker 1". The nucleic acid may have a continuous partial sequence of the sequence region. For example, the 21810279th to 21810792nd sequence region of the human chromosome 12 may be specifically the 21810279th to 21810760th sequence region, 21810279th to 21810751st sequence region, 21810450th to 21810792nd sequence region, 21810450th to 21810760th sequence region, 21810450th to 21810751st sequence region, 21810458th to 21810792nd sequence region, 21810458th to 21810760th sequence region, 21810458th to 21810751st sequence region, 21810489th to 21810792nd sequence region, 21810489th to 21810760th sequence region, 21810489th to 21810751st sequence region, 21810600th to 21810792nd sequence region, 21810600th to 21810760th sequence region, 21810600th to 21810751st sequence region, 21810750th to 21810792nd sequence region, 21810750th to 21810760th sequence region, or 21810750th to 21810751st sequence region of the human chromosome 12.

At least one CpG site comprised in the 29298021st to 29298631st sequence region of the human chromosome 17 may comprise a CpG site positioned in the 29298115th to 29298116th, 29298118th to 29298119th, 29298124th to 29298125th, 29298136th to 29298137th, 29298142nd to 29298143rd, or 29298184th to 29298185th sequences of the human chromosome 17. The 29298021st to 29298631st sequence region of the human chromosome 17 may be represented by SEQ ID NO: 2. In the present description, the nucleic acid comprising at least one CpG site comprised in the 29298021st to 29298631st sequence region of the human chromosome 17 is named "Marker 2". The nucleic acid may have a continuous partial sequence of the sequence region.

At least one CpG site comprised in the 19738547th to 19739846th of the human chromosome 19 may comprise a CpG site positioned in the 19739407th to 19739408th sequences of the human chromosome 19. The 19738547th to 19739846th of the human chromosome 19 may be represented by SEQ ID NO: 3. In the present description, the nucleic acid comprising at least one CpG site comprised in the 19738547th to 19739846th of the human chromosome 19 is named "Marker 3". The nucleic acid may have a continuous partial sequence of the sequence region.

In one embodiment of the present application, "target methylation site" is a site of interest to confirm whether the biomarker according to one embodiment of the present application is methylated or not, and may comprise at least one CpG sequence. The size of the target methylation site may be 5 to 150 bp, 5 to 140 bp, 5 to 130 bp, 5 to 120 bp, 5 to 110 bp, 5 to 100 bp, 5 to 90 bp, 5 to 80 bp, 5 to 70 bp, 5 to 60 bp, 5 to 50 bp, 5 to 40 bp, 5 to 30 bp, 5 to 20 bp, 5 to 15 bp, 5 to 10 bp, 10 to 150 bp, 10 to 140 bp, 10 to 130 bp, 10 to 120 bp, 10 to 110 bp, 10 to 100 bp, 10 to 90 bp, 10 to 80 bp, 10 to 70 bp, 10 to 60 bp, 10 to 50 bp, 10 to 40 bp, 10 to 30 bp, 10 to 20 bp, 10 to 15 bp, 20 to 150 bp, 20 to 140 bp, 20 to 130 bp, 20 to 120 bp, 20 to 110 bp, 20 to 100 bp, 20 to 90 bp, 20 to 80 bp, 20 to 70 bp, 20 to 60 bp, 20 to 50 bp, 20 to 40 bp, 20 to 30 bp, 20 to 25 bp, 30 to 150 bp, 30 to 140 bp, 30 to 130 bp, 30 to 120 bp, 30 to 110 bp, 30 to 100 bp, 30 to 90 bp, 30 to 80 bp, 30 to 70 bp, 30 to 60 bp, 30 to 50 bp, 30 to 40 bp, 30 to 35 bp, 40 to 150 bp, 40 to 140 bp, 40 to 130 bp, 40 to 120 bp, 40 to 110 bp, 40 to 100 bp, 40 to 90 bp, 40 to 80 bp, 40 to 70 bp, 40 to 60 bp, 40 to 50 bp, 40 to 45 bp, 50 to 150 bp, 50 to 140 bp, 50 to 130 bp, 50 to 120 bp, 50 to 110 bp, 50 to 100 bp, 50 to 90 bp, 50 to 80 bp, 50 to 70 bp, 50 to 60 bp, 50 to 55 bp, 60 to 150 bp, 60 to 140 bp, 60 to 130 bp, 60 to 120 bp, 60 to 110 bp, 60 to 100 bp, 60 to 90 bp, 60 to 80 bp, 60 to 70 bp, or 60 to 65 bp.

The target methylation site may comprise some or all of CpG sites present in the biomarker according to one embodiment of the present application, and as one example, may comprise a CpG site positioned in the 21810750th to 21810751st sequences of the human chromosome 12, the 29298115th to 29298116th, 29298118th to 29298119th, 29298124th to 29298125th, 29298136th to 29298137th, 29298142nd to 29298143rd, or 29298184th to 29298185th sequences of the human chromosome 17, or the 19739407th to 19739408th sequences of the human chromosome 19.

The term used in the present description, "methylation marker for a risk of developing liver cancer" means a methylation marker having a specific methylation level only in DNA isolated from a sample derived from a subject having a risk of liver cancer, compared to a methylation level of DNA isolated in a sample derived from a subject having no risk of liver cancer. For example, the methylation marker for a risk of developing liver cancer, may be a marker with a high methylation level only in DNA isolated from a sample derived from a subject having a risk of liver cancer, compared to a methylation level of DNA isolated from a sample derived from a subject having no risk of liver cancer.

The sequence represented by SEQ ID NO: 1, SEQ ID NO: 2 or SEQ ID NO: 3 may be a methylation marker for a risk of developing liver cancer. Specifically, at least one CpG site comprised in the liver cancer marker represented by SEQ ID NO: 1, SEQ ID NO: 2, or SEQ ID NO: 3 may have a high methylation level in a liver cancer-derived sample, and have a low methylation level in a sample derived from others including normal liver.

For example, when the methylated ratio of the biomarker according to one embodiment of the present application is 5% or more, 10% or more, 20% or more, 25% or more, or 30% or more in the biological sample, a subject from which the biological sample derived may be determined to have a risk of developing liver cancer. The methylated ratio may be specifically expressed as a b-value.

The agent capable of measuring a methylation level may be a primer for amplifying a target methylation site of the biomarker according to one embodiment of the present application.

The primer comprises a forward primer and a reverse primer, and one of the forward primer and the reverse primer may comprise a methylation primer and an unmethylation primer.

The methylation primer comprises at least one CpG recognition site recognizing a CpG sequence comprised in a sequence with a predetermined length comprising the target methylation site, the unmethylation primer comprises at least one TpG recognition site recognizing a TpG sequence comprised in a sequence with a predetermined length comprising the target methylation site, and the TpG sequence of the sequence with the predetermined length comprising the target methylation site is converted from the unmethylated CpG sequence of the sequence with the predetermined length comprising the target methylation site. The predetermined length may be for example, 5,000 bp or less, 4,500 bp or less, 4,000 bp or less, 3,500 bp or less, 3,000 bp or less, 2,500 bp or less, 2,000 bp or less, 1,500 bp or less, 1,400 bp or less, 1,300 bp or less, less than 1,300 bp, 1,200 bp or less, 1,100 bp or less, 1,000 bp or less, 900 bp or less, 800 bp or less, 700 bp or less, 650 bp or less, 620 bp or less, 611 bp or less, 610 bp or less, 600 bp or less, 550 bp or less, 500 bp or less, 450 bp or less, 400 bp or less, 350 bp or less, 330 bp or less, 329 bp or less, 300 bp or less, 250 bp or less, 200 bp or less, 150 bp or less, 100 bp or less, or 500 bp or less. At this time, even if the lower limit of the predetermined length is not specified, those skilled in the art can clearly set the predetermined length to measure the methylation level of the target methylation site, but it may be for example, 2 bp or more, 5 bp or more, 10 bp or more, 20 bp or more, 30 bp or more, 40 bp or more, or 50 bp or more, but not limited thereto.

The TpG sequence may be converted from an unmethylated CpG sequence by a modifying agent that modifies a methylated CpG sequence and an unmethylated CpG sequence differently from each other. For example, the modifying agent may convert an unmethylated cytosine residue into thymine. As one example, the modifying agent may be at least one selected from the group consisting of sulfurous acid, bisulfite, hydrogen sulfite and disulfite.

When a template strand, for example, a sequence represented by SEQ ID NO: 1, SEQ ID NO: 2 or SEQ ID NO: 3, is treated with a modifying agent that modifies a methylated CpG sequence and an unmethylated CpG sequence differently from each other, CpG cytosine will not be converted into thymine if CpG cytosine of the template strand is methylated, and CpG cytosine will be converted to thymine if CpG cytosine is unmethylated.

The CpG recognition site of the methylation primer may recognize a CpG sequence of a methylated nucleic acid. For example, the CpG recognition site may comprise a CG sequence.

The TpG recognition site of the unmethylation primer may recognize a TpG sequence in which a CpG sequence of an unmethylated nucleic acid is converted by a modifying agent that modifies a methylated nucleic acid and an unmethylated nucleic acid differently from each other. For example, the TpG recognition site may comprise a TG sequence.

The CpG recognition site and the TpG recognition site may be located near the 3' end of the methylation primer and unmethylation primer, respectively, and each specifically recognize a methylated nucleic acid and an unmethylated nucleic acid which are converted by a modifying agent that modifies a methylated nucleic acid and an unmethylated nucleic acid differently from each other. For example, the CpG recognition site and the TpG recognition site may be located within 40, 35, 30, 25, 20, 10, 9, 8, 7, 6, 5, 4, 3 or 2 bases from the 3' end, or at the 3' end of the methylation primer and unmethylation primer.

Since the unmethylation primer has a lower Tm value compared to the methylation primer, by adding a nucleotide at the 5' end of the unmethylation primer to supplement this, the Tm of the methylation primer and unmethylation primer may be designed similarly. For example, the unmethylation primer may further comprise a nucleotide at the 5' end, and thereby, it may have a larger size than the methylation primer. For example, the unmethylation primer may further comprise 1 to 5, 1 to 4, 1 to 3, 1 to 2, or 1 nucleotide at the 5' end compared to the methylation primer. For example, the Tm difference of the methylation primer and the unmethylation primer may be 15°C or less, 14°C or less, 13°C or less, 12°C or less, 11°C or less, 10°C or less, 9°C or less, 8°C or less, 7°C or less, 6°C or less, 5°C or less, 4°C or less, 3°C or less, 2°C or less, or 1.5°C or less.

The primer comprises a forward primer and a reverse primer, and when the methylation primer and the unmethylation primer are forward primers, the opposite direction primer is a reverse primer, and when the methylation primer and the unmethylation primer are reverse primers, the opposite direction primer is a forward primer. The opposite direction primer can bind to both a methylated nucleic acid and an unmethylated nucleic acid. Accordingly, the opposite direction primer may not comprise a CpG binding site, but when it is inevitable to comprise a CpG binding site in the opposite direction primer due to the structure of the nucleic acid, it may comprise 5 or less, 4 or less, 3 or less, 2 or less, 1 to 5, 1 to 4, 1 to 3, 1 to 2, or as one example, 1 CpG binding site. However, also at this time, it is preferable to design the opposite direction primer to have a similar Tm to the methylation primer and unmethylation primer.

The methylation primer, the unmethylation primer, and the opposite direction primer may have a size comprising 15 to 40, 15 to 35, 15 to 30, 15 to 25, 18 to 40, 18 to 35, 18 to 30, 18 to 25, 20 to 40, 20 to 35, 20 to 30, or 20 to 25 bases.

The methylation primer, the unmethylation primer, and the opposite direction primer may comprise 1 or more, 2 or more, or 3 or more Non-CpG cytosines, and may be capable of binding to nucleic acid converted by a modifying agent which modifies a methylated nucleic acid and an unmethylated nucleic acid differently from each other.

The Tm of the methylation primer, the unmethylation primer, and the opposite direction primer may be 50 to 80°C, 50 to 75°C, 50 to 70°C, 50 to 65°C, 55 to 80°C, 55 to 75°C, 55 to 70°C, 55 to 65°C, 60 to 80°C, 60 to 75°C, 60 to 70°C, or 60 to 65°C.

The composition according to one embodiment of the present application may comprise the methylation primer and the unmethylation primer at a concentration ratio of 100:1 to 1:100, 100:1 to 1:50, 100:1 to 1:20, 100:1 to 1:10, 100:1 to 1:5, 100:1 to 1:1, 100:1 to less than 1:1, 100:1 to 1.3:1, 100:1 to 1.5:1, 100:1 to 2:1, 100:1 to 2.5:1, 100:1 to 3:1, 100:1 to 3.5:1, 100:1 to 4:1, 50:1 to 1:100, 50:1 to 1:50, 50:1 to 1:20, 50:1 to 1:10, 50:1 to 1:5, 50:1 to 1:1, 50:1 to less than 1:1, 50:1 to 1.3:1, 50:1 to 1.5:1, 50:1 to 2:1, 50:1 to 2.5:1, 50:1 to 3:1, 50:1 to 3.5:1, 50:1 to 4:1, 10:1 to 1:100, 10:1 to 1:50, 10:1 to 1:20, 10:1 to 1:10, 10:1 to 1:5, 10:1 to 1:1, 10:1 to less than 1:1, 10:1 to 1.3:1, 10:1 to 1.5:1, 10:1 to 2:1, 10:1 to 2.5:1, 10:1 to 3:1, 10:1 to 3.5:1, 10:1 to 4:1, 5:1 to 1:100, 5:1 to 1:50, 5:1 to 1:20, 5:1 to 1:10, 5:1 to 1:5, 5:1 to 1:1, 5:1 to less than 1:1, 5:1 to 1.3:1, 5:1 to 1.5:1, 5:1 to 2:1, 5:1 to 2.5:1, 5:1 to 3:1, 5:1 to 3.5:1, 5:1 to 4:1, 4.5:1 to 1:100, 4.5:1 to 1:50, 4.5:1 to 1:20, 4.5:1 to 1:10, 4.5:1 to 1:5, 4.5:1 to 1:1, 4.5:1 to less than 1:1, 4.5:1 to 1.3:1, 4.5:1 to 1.5:1, 4.5:1 to 2:1, 4.5:1 to 2.5:1, 4.5:1 to 3:1, 4.5:1 to 3.5:1, or 4.5:1 to 4:1.

For example, the concentration of the unmethylation primer, may be 100% or less, less than 100%, 99.99% or less, 99.95% or less, 99.9% or less, 99.5% or less, 99% or less, 98% or less, 97% or less, 96% or less, 95% or less, 90% or less, 85% or less, 80% or less, 75% or less, 70% or less, 65% or less, 60% or less, 55% or less, 50% or less, 40% or less, 30% or less, or 25% or less of the concentration of the methylation primer, and as one example, it may be 50% or less.

Other embodiment of the present application, relates to a kit for predicting a risk of developing liver cancer, comprising the composition for predicting a risk of developing liver cancer according to one embodiment of the present application.

Other embodiment of the present application relates to a method for predicting a risk of developing liver cancer, or a method for providing information for predicting a risk of developing liver cancer, comprising a step of measuring a methylation level of at least one CpG site comprised in the 21810279th to 21810792nd sequence region of the human chromosome 12, the 29298021st to 29298631st sequence region of the human chromosome 17, or the 19738547th to 19739846th sequence region of the human chromosome 19 in a biological sample isolated from a subject.

Specifically, the method according to one embodiment of the present application may comprise a step of measuring a methylation level of at least one CpG site comprised in the 21810279th to 21810792nd sequence region of the human chromosome 12 in a biological sample, and may further comprise a step of measuring a methylation level of at least one CpG site comprised in the 29298021st to 2929863 1st sequence region of the human chromosome 17 in the biological sample; and/or a step of measuring a methylation level of at least one CpG site comprised in the 19738547th to 19739846th sequence region of the human chromosome 19 in the biological sample.

The step of measuring a methylation level may be performed by a method selected from the group consisting of PCR, methylation specific PCR, real time methylation specific PCR, MethyLight PCR, MehtyLight digital PCR, EpiTYPER, PCR using methylated DNA-specific binding protein, quantitative PCR, MS-HRM (Methylation sensitive high resolution melting), DNA chip, molecular beacon, next generation sequencing (NGS) panel, pyrosequencing and bisulfite sequencing. As one example, the step of measuring a methylation level may be performed using PCR and/or MS-HRM analysis methods using a combination of the methylation primer and unmethylation primer established in the present application.

For example, the methylation level may be identified by microarray, and the microarray may use a probe fixed on a solid surface. The probe may comprise a sequence complementary to 10 to 100 continuous nucleotide sequences comprising the CpG site.

The method according to one embodiment of the present application may further comprise a step of quantifying the methylation degree. The step of quantifying may quantify a methylation level of a biomarker comprised in the biological sample, by comparing the AUMC (Area under the melting curve) of the melting curve of a sample in which the biomarker according to one embodiment of the present application is 100% methylated, and that of a sample in which the biomarker according to one embodiment of the present application is 100% unmethylated. The melting curve may be normalized.

Specifically, the step of quantifying may comprise a step of obtaining a melt curve of the biological sample; a step of obtaining a normalized melt curve of a biological sample in which the content ratio of a methylated nucleic acid and an unmethylated nucleic acid is known; and a step of quantifying a methylation level of the biological sample, by comparing the melt curve of the biological sample and the normalized melt curve. The melt curve of the biological sample may be obtained through HRM analysis. For example, the nucleic acid may comprise at least one CpG site comprised in a nucleic acid represented by SEQ ID NO: 1, SEQ ID NO: 2, or SEQ ID NO: 3, and may be a nucleic acid having a continuous partial sequence of the nucleic acid represented by SEQ ID NO: 1, SEQ ID NO: 2, or SEQ ID NO: 3. As one example, the nucleic acid may be a nucleic acid represented by SEQ ID NO: 1, SEQ ID NO: 2 or SEQ ID NO: 3. The melt curve of the biological sample may be normalized before compared with the normalized melt curve.

The method according to one embodiment of the present application may further comprise a step of comparing the methylation level with a methylation level of a control group, after the step of measuring a methylation level. The control group means DNA isolated from a biological sample of which tissue of origin is already known, and all samples derived from a subject without liver cancer or a subject with liver cancer may be used. For example, when a sample derived from a subject with liver cancer is used as a control group, if the methylation level of DNA isolated from a biological sample is similar to the methylation level of DNA isolated from the control group, the subject from which the biological sample is derived may be determined as having a risk of developing liver cancer, and when a sample derived from a subject without liver cancer is used as a control group, if the methylation level of DNA isolated in the biological sample is higher than the methylation level of the control group, the subject from which the biological sample is derived may be determined as having a risk of developing liver cancer. The subject may be for example, a vertebrate animal, mammal, rodent, goat, deer, pig, bird, chicken, turkey, cow, horse, sheep, fish, or primate, for one example, a human.

The method according to one embodiment of the present application may further comprise a step of measuring a concentration of Alpha Fetoprotein (AFP) in the biological sample, and/or a step of collecting gender and/or age information of the subject.

The composition and method according to one embodiment of the present application may confirm whether the biomarker is methylated or not with high sensitivity, even when the concentration of the methylated biomarker is very low in the biological sample.

In order to supplement disadvantages of conventional experimental verification methods, when designing MS-HRM primers, a methylation primer comprising a CpG recognition site which recognizes a CpG sequence in the primer is used, and to prevent only a methylated template strand from being amplified, an unmethylation primer comprising a TpG recognition site which recognizes a TpG sequence to be used for amplification of an unmethylated template strand is used in combination. At this time, in order to prevent relatively low sensitivity of MS-HRM and biased amplification of the primer comprising TpG, a method to increase binding opportunity of a primer comprising CpG and reduce binding opportunity of a primer comprising TpG has been established, by adjusting the temperature of primer annealing step of PCR.

Therefore, PCR at a more sensitive level may be performed for a target present in a very trace amount such as circulating tumor DNA present in blood, for example, a nucleic acid represented by SEQ ID NO: 1, SEQ ID NO: 2 or SEQ ID NO: 3, and depending on the annealing temperature and primer concentration ratio, according to the purpose, an experiment may be designed by adjusting a degree of binding of methylation and unmethylation primers to a template strand. A design of a new experimental verification method may be constructed by supplementing disadvantages of conventional qMSP, MethyLight and MS-HRM analysis methods, and a methylation marker represented by SEQ ID NO: 1, SEQ ID NO: 2 or SEQ ID NO: 3 may be detected through liquid biopsy, and it has higher sensitivity than conventional MS-HRM analysis method and allows quantification of a methylation level.

In addition, a relative methylation ratio can be measured without control marker analysis in the same vitro, and the detection sensitivity is high as a probe interfering with amplification efficiency is not used. Furthermore, in order to measure the methylation level at a very low ratio in a biological sample such as blood and the like, the amplification efficiency of methylated and unmethylated DNA may be controlled.

The composition and method according to one embodiment of the present application use a methylation primer and an unmethylation primer in combination, so non-specific amplification products are not generated, and can measure a methylation level of a sample more sensitively than conventional PCR-based analysis methods even under a condition in which a desired target nucleic acid is present in a trace amount.

The composition and method according to one embodiment can confirm a DNA methylation level of ctDNA (circulating tumor DNA), as one example, a methylation level of at least one CpG site comprised in a nucleic acid represented by SEQ ID NO: 1, SEQ ID NO: 2 or SEQ ID NO: 3, which is present in a small amount in a cancer patient among cfDNA (cell-free DNA) present in a biological sample, for example, blood, through a semi-quantitative method in the sample, and based on this, high sensitivity and specificity can be achieved.

For example, the concentration of the methylated nucleic acid in the biological sample may be 100 ng/ul or less, 90 ng/ul or less, 80 ng/ul or less, 70 ng/ul or less, 60 ng/ul or less, 50 ng/ul or less, 40 ng/ul or less, 30 ng/ul or less, 20 ng/ul or less, 15 ng/ul or less, 10 ng/ul or less, 9 ng/ul or less, 8 ng/ul or less, 7 ng/ul or less, 6 ng/ul or less, 5 ng/ul or less, 4 ng/ul or less, 3 ng/ul or less, 2 ng/ul or less, 1.5 ng/ul or less, 1 ng/ul or less, 0.5 ng/ul or less, 0.4 ng/ul or less, 0.3 ng/ul or less, 0.2 ng/ul or less, 0.1 ng/ul or less, 0.05 ng/ul or less, 0.04 ng/ul or less, 0.03 ng/ul or less, 0.02 ng/ul or less, 0.01 ng/ul or less, 0.009 ng/ul or less, 0.008 ng/ul or less, 0.007 ng/ul or less, 0.006 ng/ul or less, 0.005 ng/ul or less, or 0.003 ng/ul or less. The nucleic acid, for example, may comprise at least one CpG site comprised in a nucleic acid represented by SEQ ID NO: 1, SEQ ID NO: 2, or SEQ ID NO: 3, and it may be a nucleic acid having a continuous partial sequence of the nucleic acid represented by SEQ ID NO: 1, SEQ ID NO: 2, or SEQ ID NO: 3. As one example, the nucleic acid may be a nucleic acid represented by SEQ ID NO: 1, SEQ ID NO: 2, or SEQ ID NO: 3.

For example, the biological sample comprises a methylated nucleic acid and an unmethylated nucleic acid, and the concentration of the methylated nucleic acid may be 75% or less, 70% or less, 60% or less, 50% or less, 40% or less, 30% or less, 25% or less, 20% or less, less than 19%, 18% or less, 15% or less, 10% or less, 5% or less, 4.5% or less, 4% or less, 3.5% or less, 3% or less, 2.5% or less, 2% or less, 1.5% or less, 1.4% or less, 1.3% or less, 1.2% or less, 1.1% or less, 1% or less, 0.9% or less, 0.8% or less, 0.7% or less, 0.6% or less, 0.5% or less, or 0.45% or less of the concentration of the unmethylated nucleic acid. For example, the nucleic acid may be a nucleic acid which comprises at least one CpG site comprised in a nucleic acid represented by SEQ ID NO: 1, SEQ ID NO: 2, or SEQ ID NO: 3, and has a continuous partial sequence of the nucleic acid represented by SEQ ID NO: 1, SEQ ID NO: 2, or SEQ ID NO: 3. As one example, the nucleic acid may be a nucleic acid represented by SEQ ID NO: 1, SEQ ID NO: 2, or SEQ ID NO: 3.

For example, the biological sample comprises a methylated nucleic acid and an unmethylated nucleic acid, and it may comprise the methylated nucleic acid of 100% or less, less than 100%, 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, less than 10%, 5% or less, less than 5%, 4.5% or less, 4% or less, 3.5% or less, 3% or less, 2.5% or less, 2% or less, 1.5% or less, 1.4% or less, 1.3% or less, 1.2% or less, 1.1% or less, 1% or less, 0.9% or less, 0.8% or less, 0.7% or less, 0.6% or less, 0.5% or less, or 0.4% or less, based on 100% of all strands of the methylated nucleic acid and the unmethylated nucleic acid. For example, the nucleic acid may be a nucleic acid which comprises at least one CpG site comprised in the nucleic acid represented by SEQ ID NO: 1, SEQ ID NO: 2, or SEQ ID NO: 3, and has a continuous partial sequence of the nucleic acid represented by SEQ ID NO: 1, SEQ ID NO: 2, or SEQ ID NO: 3. As one example, the nucleic acid may be a nucleic acid represented by SEQ ID NO: 1, SEQ ID NO: 2, or SEQ ID NO: 3.

For example, the biological sample may comprise 20,000 strands or less, 15,000 strands or less, 10,000 strands or less, 5,000 strands or less, 4,000 strands or less, 3,000 strands or less, 2,000 strands or less, 1,000 strands or less, 500 strands or less, 400 strands or less, 300 strands or less, 200 strands or less, 150 strands or less, 100 strands or less, 90 strands or less, 80 strands or less, 70 strands or less, 60 strands or less, 50 strands or less, 40 strands or less, 30 strands or less, 20 strands or less, 10 strands or less, 9 strands or less, 8 strands or less, 7 strands or less, 6 strands or less, 5 strands or less, 4 strands or less, 3 strands or less, 2 strands or less, or 1 strand or less of a methylated nucleic acid. For example, the nucleic acid may be a nucleic acid which comprises at least one CpG site comprised in the nucleic acid represented by SEQ ID NO: 1, SEQ ID NO: 2, or SEQ ID NO: 3, and has a continuous partial sequence of the nucleic acid represented by SEQ ID NO: 1, SEQ ID NO: 2, or SEQ ID NO: 3. As one example, the nucleic acid may be a nucleic acid represented by SEQ ID NO: 1, SEQ ID NO: 2, or SEQ ID NO: 3.

In one embodiment of the present application, the biological sample may comprise one or more selected from the group consisting of blood, plasma, serum, platelets, tissue, cells, feces and urine.

In one embodiment of the present application, the liver cancer may be hepatocellular carcinoma.

The method according to one embodiment of the present application may further comprise a step of treating a subject. The step of treating may comprise a step of performing administration of an effective amount of a therapeutic agent, chemotherapy, hormone therapy, radiotherapy, surgery, or a combination thereof for the subject. The therapeutic agent may be for example, a liver cancer therapeutic agent.

The therapeutic agent, for example, may include Afatinib, AK105, Anlotinib, Apatinib, Atezolizumab, Avelumab, axitinib, Bevacizumab, bosutinib, BSC, Cabozantinib, Cabozantinib-S-Malate, Camrelizumab, canertinib, carboplatin, capecitabine, celecoxib, CC-122, CF102, crizotinib, dasatinib, docetaxel, Donafenib, Dovitinib, doxorubicin, Durvalumab, EKB-569, entrectinib, epirubicin, erlotinib, etoposide, everollmus, FGF401, FOLFOX 4, fostamatinib, Galunisertib, gefitinib, gemcitabine, IBI305, ibrutinib, imatinib, INC280, Infigratinib, Ipilimumab, irinotecan, lapatinib, leflunomide, Lenvatinib, LY2875358, Mesylate, mitomycin c, MSC2156119J, neratinib, nilotinib, Nintedanib, Nivolumab, oxaliplatin, Palbociclib, Panobinostat, pazopanib, PDR001, Pembrolizumab, Pemigatinib, Pexavec, Phosphate, Ramucirumab, Regorafenib, ruxolitinib, semaxinib, selumetinib, SGO-110, SHR-1210, Sintilimab, Sorafenib, SU6656, sunitinib, sintilimab, spartalizumab, sutent, TACE, Tasquinimod, Temozolomide, Temsirolimus, Tislelizumab, Tivantinib, Tosylate, toripalimab, Tremelimumab, vandetanib, vatalanib, XL888, Y90, a pharmaceutically acceptable salt thereof, or a combination thereof.

Other embodiment of the present application relates to a method for treating liver cancer, comprising a step of treating a subject determined to have a risk of liver cancer according to one embodiment of the present application. The step of treating a subject is as described above.

[ADVANTAGEOUS EFFECTS]

The biomarker according to one embodiment of the present application can achieve much higher sensitivity by diagnosis through liquid biopsy than conventionally used liquid biopsy diagnosis methods, and through this, the efficiency of liver cancer tests increases exceptionally to significantly increase the survival rate of liver cancer patients.

In addition, one embodiment of the present application can provide a biomarker capable of liver cancer-specific early diagnosis through a blood-based liquid biopsy method and a molecular test method using the same. In particular, by confirming a DNA methylation level of blood-derived cell-free DNA (cfDNA), presence or absence of cfDNA derived from a liver cancer cell of a test subject can be detected.

[BRIEF DESCRIPTION OF THE DRAWINGS]

FIG. 1 is a drawing which confirms a methylation level in each tissue of the marker according to one embodiment of the present application, and in the horizontal axis, it means that CGRC_N: Cancer Genome Research Center normal; LIHC_N: Liver Hepatocellular carcinoma normal, CGRC_T: Cancer Genome Research Center tumor, and LIHC_T: Liver Hepatocellular carcinoma tumor.

FIG. 2 is a drawing which shows the methylation distribution of the marker according to one embodiment of the present application in normal liver and liver disease, and in the horizontal axis, it means that Liver_N: normal liver; NAFLD: Non-alcoholic fatty liver disease; NASH: Non-alcoholic steatohepatitis; LGDN: low-grade dysplastic nodule; HGDN: high-grade dysplastic nodule; HCC: hepatocellular carcinoma; HepG2: hepatocellular carcinoma (HCC) cell line.

FIG. 3a to FIG. 3c are drawings which show the results of verifying the performance of the biomarker according to one embodiment of the present application using universal methylation data as an independent cohort.

FIG. 4 is a drawing which shows liver cancer specificity of the biomarker according to one embodiment of the present application, and the X-axis represents normal and cancer tissue samples of 23 carcinoma sets including liver cancer, and the Y-axis represents the biomarker, and the color of the matrix represents the methylation level.

FIG. 5a to FIG. 5c are the results of confirming the possibility of distinguishment of the methylated and unmethylated strands of the method for measuring the methylation level of the biomarker according to one embodiment of the present application, by using Control DNA (EpiTect PCR Control DNA Set, Qiagen, 59695), gDNA of the liver cancer cell line and sulfite-converted gDNA derived from human Buffy Coat.

FIG. 6a to FIG. 6c are the results of analyzing the minimal limit of detection for methylated and unmethylated DNA strands of Marker 1 which is the biomarker according to one embodiment of the present application, by using the method for measuring the methylation level according to one embodiment of the present application.

FIG. 7 is a boxplot showing the methylation level of the biomarker according to one embodiment of the present application for normal and tumor tissue samples of various carcinomas.

FIG. 8a to FIG. 8c are drawings which show the results of the MS-HRM analysis method based on sulfite-converted gDNA derived from Buffy Coat collected from 55 healthy people, FIG. 8a and FIG. 8b are drawings which show normalized melting point curves of all specimens, and FIG. 8c is a boxplot showing the distribution of the integral value (AUMC) of each sample based on the melting point curve.

FIG. 9a is a boxplot showing the AUMC of 81 healthy people and 319 HCC patients, and the cut-off set based on the 97th Percentile of the AUMC value of the 81 healthy is shown as a dotted line.

FIG. 9b is the results of analyzing liver cancer diagnosis accuracy of Marker 1 which is the biomarker according to one embodiment of the present application for a total of 420 clinical specimens.

FIG. 10 is a drawing which shows the results that the accuracy of liver cancer diagnosis increases when Marker 1, which is the biomarker according to one embodiment of the present application, is combined with various biomarkers.

FIG. 11 is a drawing which confirms liver cancer specificity of the diagnosis marker according to one embodiment of the present application.

FIG. 12 is a drawing which confirms predicting a risk of developing liver cancer accurately through the methylation level of the diagnosis marker according to one embodiment of the present application.

[MODE FOR INVENTION]

Hereinafter, the present application will be described in more detail by the following Examples. However, these Examples are intended to illustrate the present application only, but the scope of the present application is not limited by these Examples.

**Example 1. Discovery of markers for predicting a risk of developing liver cancer**

In order to discover markers for predicting a risk of developing liver cancer capable of blood biopsy, DNA methylation data of the TCGA (The Cancer Genome Atlas) database and CGRC (Cancer Genome Research Center) were analyzed. By analyzing the methylation data of the normal liver tissue and liver cancer tissue of the database, CpG sites which were low methylated by 15% or less in the normal liver sample of 95% or more, also low methylated by 15% or less in the blood sample of 95% or more, and highly methylated by 30% or more in the liver cancer sample, were selected.

To discover universal biomarkers, machine learning was performed for each data set of the database to find CpG sites commonly with a high machine learning importance ranking.

In order to discover markers capable of blood biopsy, a marker candidate group which was low methylated by 15% or less in normal tissue of organs other than liver was additionally discovered.

The information of the marker selected as satisfying all the above conditions was shown in Table 1. The selected biomarker was named "Marker 1" in the present description.

In addition, 2 kinds of markers shown to be specific to liver cancer were additionally selected, and named "Marker 2" and "Marker 3", respectively, and the information was shown in Table 1.

[Table 1]

| Name | Chromosome | Start | End | Sequence | SEQ ID NO: |
|---|---|---|---|---|---|
| Marker 1 | Chr12 | 21810279 | 21810792 | CGAGGTAAAGATCGTCCAGCCTTCATAGGCCACCTGGTTCCGTCCCCCTCCCCCAGCTACTCGGGCACGTGTCCCGACTCCCGCCAGCAGGCCGTGGGGGTGATGTGGCCGCCATGAACCCACCAAGGACAAGTAGGGCCTGGTTTTAGCTGCAAATGCAACAAGTCTCTCCGCTGAGCCGCAGCTGCTACCCTCTGCCTTCTGCTCCTCCGCCAGGGCACGGCCCCCCCTGCGCCCCAAACTGAGCGGCAAAGTCAGGGCCCGCGGCCGGATGCTCAGAGCTAAAGGCCGCGGAGGACAGATGTGCTTCTTCCTCCTTCCCGCGTCTCCCCATACAAGTACTACCCCGCACGTCCCATCAGGCTTGCCTGTGGGCCAGGATTCAGGGTCCTGAGCCGAAACCTACCAGGAGAGAGAAGGCTCTGGAGACCTCTGTAACAGTCGTGCGGAGAAGACAAAGTCAGCTGCGTG**C****G**TCTCCTCCGGCGCCGGCTCTGCAAGGAGGGAGGAGGGGGCG | 1 |
| Marker 2 | Chr 17 | 29298021 | 29298631 | GGGCGAGGGAGGAGCCTGAGGAGACTCGCCCGGCTCAACCCCGACGTCCGCGCCCCGGCCGCCTGTTGGCCATGGCGGGCCTGGGCCTGGGCTCCGCCGTTCC**CG**TGTGGCTGGCCGAGGACGACCTCGGCTGCATCATCTGCCAGGGGCTGCTGGACTGGCCCGCCACGCTGCCCTGCGGCCACAGCTTCTGCCGCCACTGCCTGGAGGCCCTGTGGGGCGCCCGCGACGCCCGCCGCTGGGCCTGCCCCACTTGCCGCCAGGGCGCCGCGCAGCAGCCGCACCTGCGGAAGAACACGCTACTGCAGGACCTGGCCGACA | 2 |

(continued)

| Name | Chromosome | Start | End | Sequence | SEQ ID NO: |
|------|-----------|-------|-----|----------|------------|
|  |  |  |  | AGTACCGCCGCGCCGCACGCG AGATACAGGCGGGCTCCGACC CTGCCCACTGCCCCTGCCCGGG CTCCAGTTCCCTCTCCAGCGCG GCCGCGAGGCCCCGGCGCCGC CCGGAACTGCAGCGGGTAGGG AGGCCGGGCCCGCAGCTCCCC TGGCTCCCCGGGCTGCCCGCC GCCTGACCCTTTCCCATGTGGC TCGAACCCCTTTCCTCAGCCGT TCTACTTTTACGTTCCTTTTCTC AGTCTAAAAGTCGAGTTCCGCT CTTCGGAGGCACTTTGGAAAGT TCATAAAA |  |

(continued)

| Name | Chromosome | Start | End | Sequence | SEQ ID NO: |
|------|-----------|-------|-----|----------|-----------|
| Marker 3 | Chr 19 | 19738547 | 19739846 | GGGTGAGGTCACTTGCTAGAA CCCTGCGAGGGGGCGGTGCCG AGGCGTGGAGGTGATCGGAGT GGACAGAGAGGCGGCCCCAGA AGTTAGTCGGCGGGATTTGGG CGCGAGTGCTTCCCAAGCGGG CGGGGCAATAGGCTGTTCCGG GTGGGACGGAGGACACTGGCG GTCTCCGGGACAAAGACAGCG GTTGGGGAAGGGATGGATGGC CGGGCCTCCAGGCGTCTCCAG ACTGGGGAAGGGGTAGGGGGT TAAGGGATCAGAGGGCGGCGT CCTGGCCCCGCAGGCACGTGC CGCGCCCTTCCCCCGGGCCCAG GCCCGCACCCGCCCGCCTCCCG TCCGTGCAGGCCTCACCTGGGC CTCCAGTCACGCCGCAGACAA GGCGGGCTCGCGGGGGTTCGG GGCGGGCTCGAGGCCCATGGC CCGGCGACTGCGGCGGGAGCG GTAGAGCGTCCGGGTGCGCCC TGTGCGGGTTGCTGAGAGGGC GCGGGAGGCGGGGTCTCCCCG GCGCCCCGCCCCCACCACCTGG GGGAGCCCTGCCCCGCCCCGC CACCTGGGGGAGCCCCGCCTC CACGGCCGCGTCGGGGGCGGT GTCGCCCGGAACCGCAGCCCC CCTGTTTCCTCCCCGCCTCGCG GCCCCTCAGTCGCCCCTATTTC CGGCGCGGGACCCCCACCCCG GTACCTGCACACCTCATGCGCC CGCAGTTTCGAGCCTGCGGGG | 3 |

(continued)

| Name | Chromosome | Start | End | Sequence | SEQ ID NO: |
|---|---|---|---|---|---|
| | | | | CTGAGTCACTCGGCGACCCGA AAATTCCCGCCGTGACCCCGCG CTGCCCGGGCGGAGCTCCAGG TGGTCTTGGGTCCGCGCCCCCG CTCCGGACCCCCAACCCTCCCA GCCCCACCCCGCTCTCTTCTTC GTGTCCAAT**CG**GCCGCCGAGT TACAAGCCACTCCCTGATCCGA CTTGGCTCCCACCGCCCGGACC CCCGCCCAGCTTCGGCTTTGCC AGCTCGCAGTATCAGCGAGCC GCGGCCTCCTGTGCGAGGCCC ACCTCTGCGGCCCCAGCTGATC ATCGGGCGCACGCTAGGGTTTT GCAATCCCGCCCCCGTACCCCT CACCCTGGGACAGCGCTGCCC CCTGCAAAGCCCGGCCCTGCCC TGCCCTGCCCTGTGGCTTCCTC CCTCGGCCTCCCTGCGGCGGCC CCGGGCTCCCACGGTCCGGAG CACACAGCCCCATTGTCCCGGT TATCGGTACTTTGTTATACTGT ATTTGATTATGCAGGACCGGAT CGGGGACGCAGAGCTCCGGGA GGGCCGGGCTGGCGTGACTCA GCCGGGTGCTGCGACCCAGCT CCAACTGCCTTTACGTA | |

In the following Examples, as one example of Marker 1, the CpG site positioned in the 21810750th to 21810751st sequences of the chromosome 12 was representatively used for the experiment, as one example of Marker 2, the CpG site positioned in the 29298124th to 29298125th sequences of the chromosome 17 was representatively used for the experiment, and as one example of Marker 3, the CpG site positioned in the 19739407th to 19739408th sequences of the chromosome 19 was representatively used for the experiment. In the sequences of Table 1, the CpG site positioned in the 21810750th to 21810751st sequences of the chromosome 12, the CpG site positioned in the 29298124th to 29298125th sequences of the chromosome 17, and the CpG site positioned in the 19739407th to 19739408th sequences of the chromosome 19 were indicated in bold and underlined.

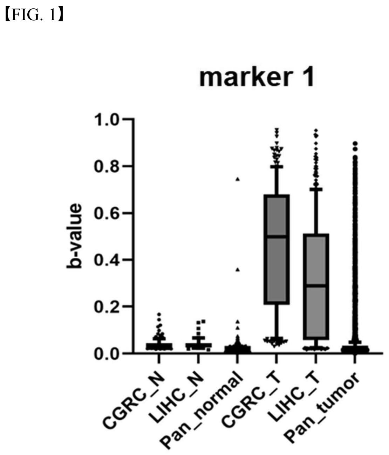

The selected liver cancer markers were visualized by a box plot using Graph pad prism tool and were shown in FIG. 1 (X-axis: sample type; CGRC_N: Cancer Genome Research Center normal; LIHC_N: Liver Hepatocellular carcinoma normal; CGRC_T: Cancer Genome Research Center tumor; LIHC_T: Liver Hepatocellular carcinoma tumor, Y-axis: methylation B-value).

As shown in FIG. 1, the marker according to one embodiment of the present application were low methylated in normal blood, normal liver tissue, normal tissue of organs other than liver, and cancer tissue of organs other than liver, and showed a characteristic of being highly methylated specifically in the liver cancer sample. Specifically, the marker according to one embodiment of the present application showed 30% or more of the mean methylation value in the liver cancer sample, while the methylation level was significantly low in other samples, thus it could specifically distinguish a liver cancer sample through the methylation level of the marker.

**Example 2. Methylation alternation according to liver cancer developing stage**

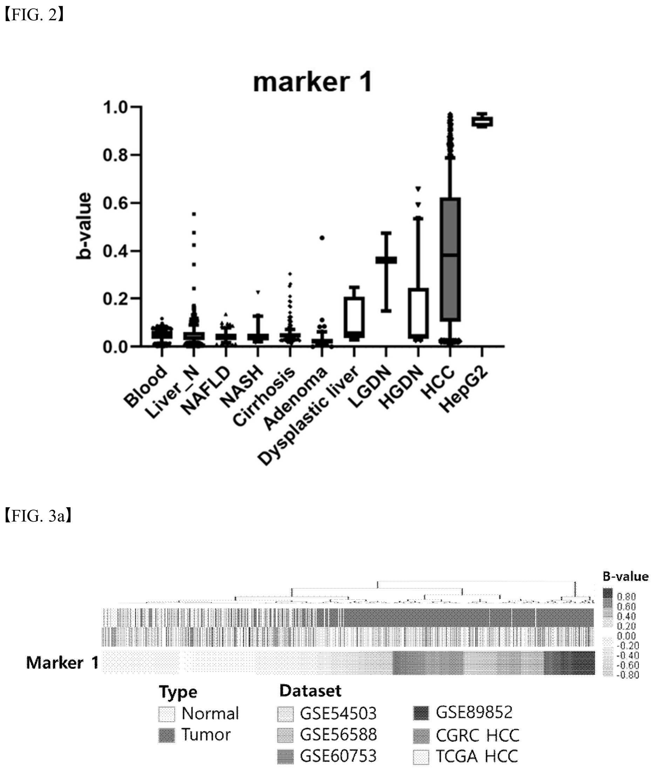

In order to identify methylation alternation according to the developing stage of liver cancer for the biomarker according to one embodiment of the present application, data sets in the developing stage of cancer were downloaded from GEO (Gene Expression Omnibus) database. Various methylation data at each stage were combined and visualized as a box plot using Graph pad prism tool and shown in FIG. 2 (X-axis: sample type; Liver_N: normal liver; NAFLD: Non-alcoholic fatty liver disease; NASH: Non-alcoholic steatohepatitis; LGDN: low-grade dysplastic nodule; HGDN: high-grade dysplastic nodule; HCC: hepatocellular carcinoma; HepG2: hepatocellular carcinoma (HCC) cell line, Y-axis: methylation B-value).

As shown in FIG. 2, the biomarker according to one embodiment of the present application showed a pattern of low methylation in the normal blood, normal liver tissue, fatty liver, and hepatocirrhosis, the methylation gradually increases from dysplastic liver and dysplastic nodule stage which is a precancerous lesion of liver cancer, and it showed significantly high methylation levels in the hepatocellular carcinoma (HCC). In FIG. 2, the X-axis represents the specimen, and the Y-axis represents the methylation value.

**Example 3. Verification of clinical performance of biomarkers in independent cohorts**

In order to verify the biomarkers according to one embodiment of the present application in various independent cohorts, 4 liver cancer methylation data sets, GSE54503, GSE56588, GSE60753, and GSE89852 were downloaded from GEO (Gene Expression Omnibus) database and used for an experiment. By combining the 2 data sets (CGRC HCC, TCGA HCC) used for marker discovery in Example 1 and the above 4 data sets, hierarchical clustering was performed using the biomarkers according to one embodiment of the present application, and this was visualized and shown in FIG. 3a to FIG. 3c (X-axis: sample, Y-axis: Probe ID of marker, green box: normal sample, red box: cancer sample).

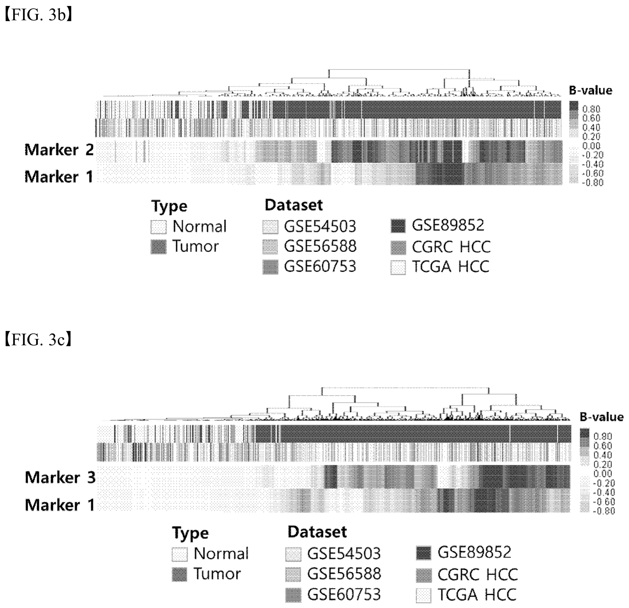

FIG. 3a is the clustering result of the single marker of Marker 1 according to one embodiment of the present application, and FIG. 3b is the clustering result of the combination marker of Marker 1 and Marker 2, and FIG. 3c is the clustering result of the combination marker of Marker 1 and Marker 3. In FIG. 3a to FIG. 3c, the color of the heatmap indicates the methylation level, and the color from white to red indicates high methylation. As shown in FIG. 3a to FIG. 3b, as a result of clustering all the samples of the 6 data sets with the heatmap, it could be confirmed that the normal sample indicated in green and the liver cancer sample indicated in pink were distinguished. In addition, as a result of performing hierarchical clustering by applying Marker 1, and Marker 2 or Marker 3 according to one embodiment of the present application together, tumor coverage was further improved.

**Example 4. Verification of liver cancer specificity of biomarkers**

In order to confirm the liver cancer specificity of the biomarkers according to one embodiment of the present application, the mean methylation level was analyzed by cancer type using the methylation data of the TCGA and GEO databases used in Example 1 or Example 2.

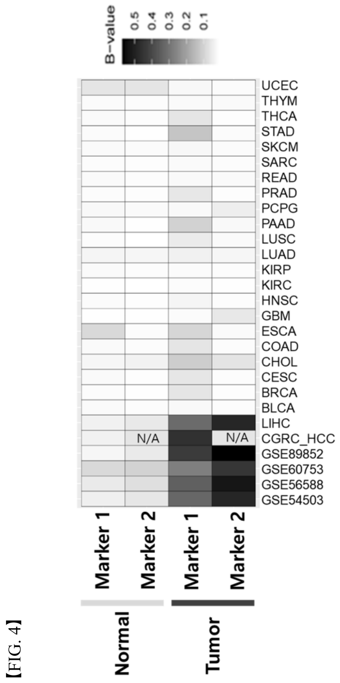

Specifically, the methylation level of Marker 1 and Marker 2 by each data set was confirmed with a heatmap by using ggplot2 R package in R software, and shown in FIG. 4 (blue: low methylation, red: high methylation, X-axis: sample, Y-axis: Probe ID of marker, green box: normal sample, red box: cancer sample).

As shown in FIG. 4, as a result of confirming the mean methylation value of the biomarkers according to one embodiment of the present application in each cancer type, Marker 1 and Marker 2 were shown in red only in the liver cancer specimen, confirming highly methylated pattern, and they were shown in blue in normal liver and other cancer types, confirming a low methylated pattern. Therefore, as both the 2 biomarkers showed a high methylation level only in the liver cancer, and showed a low methylation level in other cancer types, the liver cancer specificity was verified.

**Example 5. Establishment of method for measuring methylation level of biomarkers**

A method for measuring a methylation level of the biomarkers according to one embodiment of the present application was established as follows.

Various samples comprising Marker 1 according to one embodiment of the present application and a control group sample were prepared as Table 2 and used for an experiment. Samples with sample numbers 2, 3 and 5 were purchased from Qiagen and used, and in sample number 3, gDNA of sample number 2 was treated with sulfite using EpiTect Bisulfite Kit to convert all cytosine residues into thymine, and in sample number 5, gDNA of sample number 2 was 100% methylated using SssI methylase and then sulfite conversion treatment was performed using EpiTect Bisulfite Kit to convert cytosine other than CpG cytosine into thymine. In case of sample number 4, gDNA was extracted using QIAmp DNA Blood Kit (Qiagen, Cat. No. 51104) from peripheral blood mononuclear cells using a buffy coat isolated from blood of a healthy human according to the manufacturer's instructions, and for the extracted gDNA, sulfite conversion was performed using EZ DNA Methylation-Lightning Kit, and it was used for an experiment. In case of sample number 6, gDNA of Huh-1, a liver cancer cell line, was prepared to be utilized for an experiment using QIAmp DNA Blood Kit and EZ DNA methylation-Lightning Kit as same as PBMC. Each sample was prepared by measuring the concentration by Qubit, and then diluted to 0.33 ng/μL.

[Table 2]

| Sample number | Sample name | Sample description | Methylated or not | Graph color of FIG. 5 |
|---|---|---|---|---|
| 1 | NTC | non-template control | - | Black |
| 2 | EpiTect gDNA | Sulfite untreated sample | - | Green |
| 3 | EpiTect Unmet. DNA | 100% unmethylated sample | X | Purple |
| 4 | Buffy Coat gDNA | Normal blood cell-derived sample | X | Orange |
| 5 | EpiTect Met. DNA | 100% methylated sample | O | Blue |
| 6 | Huh-1 gDNA | liver cancer cell-derived sample | O | Red |

The sulfite converted sequences of the biomarkers according to one embodiment of the present application were shown in Table 3. In Table 3, C in which CT conversion occurred by sulfite treatment was indicated with a lowercase letter t, and the methylated CpG site was indicated with an uppercase letter CG. In Table 3, the CpG sites used for the experiment representatively in the Example of the present application was indicated in bold and underlined.

[Table 3]

| name | Bisulfite converted Sequence | SEQ ID NO: |
|---|---|---|
| Marker 1 | CGAGGTAAAGATCGTttAGttTTtATAGGttAttTGGTTtCGTtttttTttttAGtTAtTCGGGtACGTGTttCGAtTttCGttAGtAGGtCGTGGGGGTGATGTGGtCGttATGAAtttAttAAGGAtAAGTAGGGttTGGTTTTAGtTGtAAATGtAAtAAGTtTtTtCGtTGAGtCGtAGtTGtTAtttTtTGttTTtTGtTttTtCGttAGGGtACGGttttttttTGCGttttAAAtTGAGCGGtAAAGTtAGGGttCGCGGtCGGATGtTAGAGtTAAAGGtCGCGGAGGAtAGATGTGtTTtTTttTttTTttCGCGTtTttttATAtAAGTAtTAtttCGtACGTtttATtAGGtTTGttTGTGGGttAGGATTtAGGGTttTGAGtCGAAAttTAttAGGAGAGAGAAGGtTtTGGAGAttTtTGTAAtAGTCGTGCGGAGAAGAtAAAGTtAGtTGCGTG**CG**TtTttTtCGGCGtCGGtTtTGtAAGGAGGGAGGAGGGGGCG | 4 |
| Marker 2 | GGGCGAGGGAGGAGttTGAGGAGAtTCGttCGGtTtAAtttCGACGTtCGCGtttCGGtCGttTGTTGGttATGGCGGGttTGGGttTGGGtTtCGtCGTTtt**CG**TGTGGtTGGtCGAGGACGAttTCGGtTGtATtATtTGttAGGGGtTGtTGGAtTGGttCGttACGtTGtttTGCGGttAtAGtTTtTGtCGttAtTGttTGGAGGtttTGTGGGGCGttCGCGACGttCGtCGtTGGGttTGttttAtTTGtCGttAGGGCGtCGCGtAGtAGtCGtAttTGCGGAAGAAtACGtTAtTGtAGGAttTGGtCGAtAAGTAtCGtCGCGtCGtACGCGAGATAtAGGCGGGtTtCGAtttTGtttAtTGttttTGttCGGGtTttAGTTttttTtTttAGCGCGGtCGCGAGGtttCGGCGtCGttCGGAAtTGtAGCGGGTAGGGAGGtCGGGttCGtAGtTttttTGGtTttttCGGGtTGttCGtCGttTGAtttTTTtttATGTGGtTCGAAttttTTTttTtAGtCGTTtTAtTTTTACGTTttTTTTtTtAGTtTAAAAGTCGAGTTtCGtTtTTCGGAGGtAtTTTGGAAAGTTtATAAAA | 5 |
| Marker 3 | GGGTGAGGTtAtTTGtTAGAAtttTGCGAGGGGGCGGTGtCGAGGCGTGGAGGTGATCGGAGTGGAtAGAGAGGCGGttttAGAAGTTAGTCGGCGGGATTTGGGCGCGAGTGtTTtttAAGCGGGCGGGGtAATAGGtTGTTtCGGGTGGGACGGAGGAtAtTGGCGGTtTtCGG | 6 |

(continued)

| name | Bisulfite converted Sequence | SEQ ID NO: |
|---|---|---|
| | GAtAAAGAtAGCGGTTGGGGAAGGGATGGATGGtCGGGttTttA GGCGTtTttAGAtTGGGGAAGGGGGTAGGGGGGTTAAGGGATtAG AGGGCGGCGTttTGGtttCGtAGGtACGTGtCGCGtttTTttttCGGGttt AGGttCGtAttCGttCGttTttCGTtCGTGtAGGtTtAttTGGGttTttAGTtA CGtCGtAGAtAAGGCGGGtTCGCGGGGGGTTCGGGGCGGGtTCG AGGtttATGGttCGGCGAtTGCGGCGGGAGCGGTAGAGCGTtCGG GTGCGtttTGTGCGGGTTGtTGAGAGGGCGCGGGAGGCGGGGTt TtttCGGCGtttCGttttAttAttTGGGGGAGtttTGtttCGtttCGttAttTGGG GGAGtttCGttTttACGGtCGCGTCGGGGGCGGTGTCGttCGGAAtC GtAGtttttTGTTTttTtttCGttTCGCGGttttTtAGTCGttttTATTTtCGGC GCGGGAtttttAtttCGGTAttTGtAtAttTtATGCGttCGtAGTTTCGAGtt TGCGGGGtTGAGTtAtTCGGCGAttCGAAAATTttCGtCGTGAtttC GCGtTGttCGGGCGGAGtTttAGGTGGTtTTGGGTtCGCGttttCGtTt CGGAttttttAAtttTtttAGttttAtttCGtTtTtTTtTTCGTGTttAAT**CG**tCGt CGAGTTAtAAGttAtTtttTGATtCGAtTTGGtTtttAtCGttCGGAttttCGt ttAGtTTCGGtTTTGttAGtTCGtAGTATtAGCGAGtCGCGGttTttTGT GCGAGGtttAttTtTGCGGttttAGtTGATtATCGGGCGtACGtTAGGG TTTTGtAATttCGttttCGTAttttTtAtttTGGGAtAGCGtTGttttttTGtAAA GttCGGtttTGtttTGtttTGtttTGTGGtTTttTtttTCGGttTtttTGCGGCGGtt tCGGGtTtttACGGTtCGGAGtAtAtAGttttATTGTttCGGTTATCGGT AtTTTGTTATAtTGTATTTGATTATGtAGGAtCGGATCGGGGAC GtAGAGtTtCGGGAGGGtCGGGtTGGCGTGAtTtAGtCGGGTGtTG CGAtttAGtTttAAtTGttTTTACGTA | |

*t: C in which CT conversion occurs by sulfite treatment
*CG: methylated CG

The size of the PCR amplification products was set to be 500 to 100 bp, and considering that the PCR heating cooling (annealing) temperature was about 60°C, the Tm of the primer was set to be 60 to 65°C. To specifically bind to one of methylated or unmethylated strands, CpG or TpG sequence was to be comprised near the 3'-end of either primer of the forward or reverse one. Since the CpG cytosine remained without being converted into thymine after sulfite treatment in the methylated template strand, the methylation primer was designed to comprise CpG as it is, and the unmethylation primer was designed to comprise TpG. At this time, the primer specifically binding to the unmethylated template strand had a lower Tm than the primer specifically binding to the methylated template strand, so in order to supplement this, the Tm of the two kinds of primers can be designed similarly by adding a nucleotide to the 5'-end. Primer in the opposite direction to the methylation primer and unmethylation primer was designed as a primer capable of binding regardless of the methylation state of the methylated strand and unmethylated strand. All primers comprised 2 or more Non-CpG cytosines to enable specific binding to sulfite converted DNA strands. The exemplary sequences of the primer set produced for the Marker 1 which is the biomarker according to one embodiment of the present application, were shown in Table 4. In each primer sequence, the sites corresponding to CpG or Non-CpG cytosine of Marker 1 were written in uppercase letters, and other bases were written in lowercase letters.

[Table 4]

| name | Seq (5'->3') | SEQ ID NO. |
|---|---|---|
| Methylated Forward Primer for Marker 1 | aAcCGACGcCGAaAAaAaCG | 7 |
| Methylated Reverse Primer for Marker 1 | gaaggTtTtggagaTTtTtgtaaTag | 8 |
| Unmethylated Forward Primer for Marker 1 | ttAcaAaAcCAACAcCAAaAAaAa CA | 9 |

Using the primers of Table 4, PCR Mixture was prepared as follows (based on 1 rxn):

Master Mix (2X): 12.5 μL (Final Conc. 1X)
EvaGreen (20X): 1.25 μL (Final Conc. 1X)
Methylated Forward Primer (10 uM): 1 μL (Final Conc. 0.4 uM)
Methylated Reverse Primer (10 uM): 1 μL (Final Conc. 0.4 uM)
Unmethylated Forward Primer (10 uM): 0.5 μL (Final Conc. 0.2 uM)
NFW (nuclease-free water): 5.75 μL
Total: 22 μL

The prepared PCR Mixture was aliquoted by 22 μL in each well of each 96-well plate, and then the control nucleic acid sample at a concentration of 0.33 ng/μL was aliquoted in each well by 3 μL. PCR reaction and HRM analysis were performed under the following conditions:

95°C 5 min / 95°C 20 sec - 69°C 40 sec (20 Cycle, starting at 69°C, decreasing by 0.3°C per Cycle, and ending at 63°C): PCR reaction
95°C 20 sec - 63°C 40 sec (40 Cycle, progressing constantly at 63°C): PCR reaction
63°C 5 min / 60°C - 95°C (measuring a fluorescence value, after reaction for 10 seconds by increasing by 0.2°C): HRM analysis

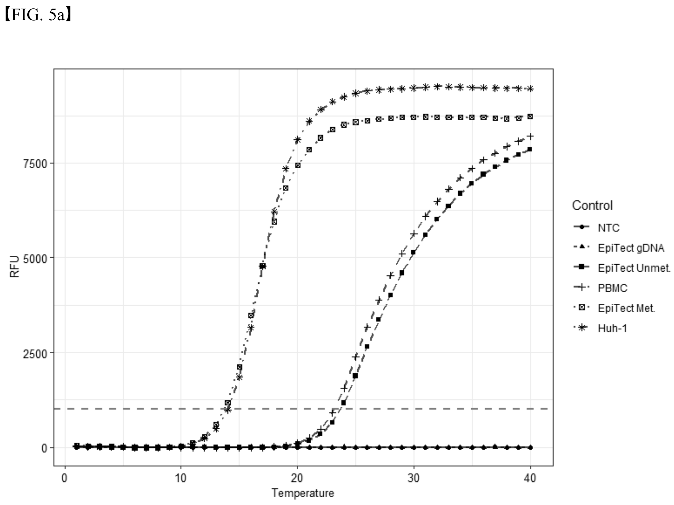

The fluorescence value for the PCR and HRM analysis was confirmed, and a melting curve or peak was confirmed, thereby confirming characteristics of the methylated and unmethylated control groups. The result of the PCR and HRM analysis for Marker 1 according to one embodiment of the present application was shown in FIG. 5a to FIG. 5c.
FIG. 5a is a drawing observing fluorescence signals in the PCR step during the MS-HRM analysis process, and only the signals for the methyl sulfite-converted specimens of the samples during the MS-HRM analysis were confirmed. Specifically, through the NTC and EpiTect gDNA results, the primers for the biomarker according to one embodiment of the present application did not form a dimer through a reaction between the primers, and did not react with gDNA in which sulfite conversion was not conducted, and did not produce PCR amplification products. Therefore, it was confirmed that the designed primers reacted specifically only to the sulfite-converted specimens.

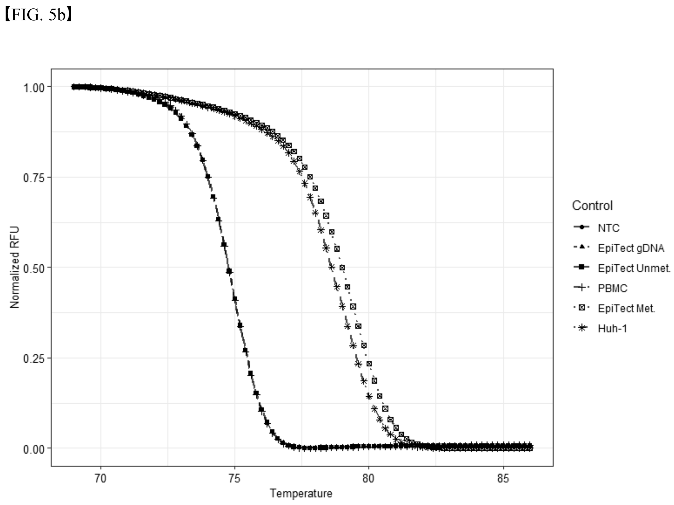

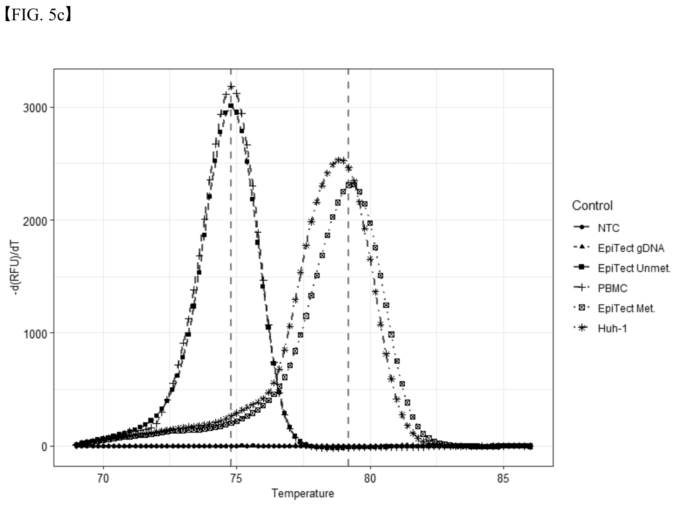

FIG. 5b is a melt curve showing that double strands of the PCR amplification products are decomposed due to the temperature increase after the PCR reaction, and FIG. 5c is a melt peak graph showing -(differential value) for the melt curve of FIG. 5b. In case of the methylated template strands, CpG was maintained as it is, and denaturation of the PCR amplification products occurred at a relatively higher temperature than the unmethylated template strands modified into TpG. In other words, it could be confirmed that the double strands of the PCR amplification products produced by unmethylated DNA were decomposed into single strands at a lower temperature than the PCR amplification products produced by methylated DNA. Accordingly, it can be seen that the primers designed by targeting the biomarker according to one embodiment of the present application can reflect characteristics of the amplification products depending on the methylation level of the sample.

**Example 6. Minimal limit of detection (LoD)**

**(1) Analysis of minimal limit of detection (1)**

The concentration of the control nucleic acid sample to be used (sulfite-converted gDNA of Buffy Coat and Huh-1) was measured through Qubit, and then it was prepared by diluting it to 1 ng/μL. The control nucleic acid of 1 ng/μL was continuously diluted with water by 2 folds, and thereby, each sample was prepared to a concentration of 0.016 ng/μL (1 ng/μL, 0.5 ng/μL, 0.25 ng/μL, 0.13 ng/μL, 0.06 ng/μL, 0.03 ng/μL, and 0.016 ng/μL, total of 7 concentrations of control nucleic acids).
Using the primers of Table 4, PCR Mixture was prepared as follows (based on 1 rxn):

Master Mix (2X): 12.5 μL (Final Conc. 1X)
EvaGreen (20X): 1.25 μL (Final Conc. 1X)
Methylated Forward Primer (10 uM): 1 μL (Final Conc. 0.4 uM)
Methylated Reverse Primer (10 uM): 1 μL (Final Conc. 0.4 uM)
Unmethylated Forward Primer (10 uM): 0.5 μL (Final Conc. 0.2 uM)
NFW (nuclease-free water): 5.75 μL
Total: 22 μL

The prepared PCR Mixture was aliquoted by 22 μL in each well of each 96-well plate, and then the control nucleic acid sample at a concentration of 0.33 ng/μL was aliquoted in each well by 3 μL. PCR reaction and HRM analysis were performed under the following conditions:

95°C 5 min / 95°C 20 sec - 69°C 40 sec (20 Cycle, starting at 69°C, decreasing by 0.3°C per Cycle, and ending at 63°C): PCR reaction
95°C 20 sec - 63°C 40 sec (40 Cycle, progressing constantly at 63°C): PCR reaction
63°C 5 min / 60°C - 95°C (measuring a fluorescence value after reaction for 10 seconds by increasing by 0.2°C): HRM analysis

The correlation between PCR Cq values depending on the Input concentration was confirmed, and the minimal limit of detection (LoD) was verified. Through AUMC (area under the melting curve) based on a normalized melting point curve in all concentration sections, whether the PCR amplification products of the methylated and unmethylated template strands formed correctly was verified.

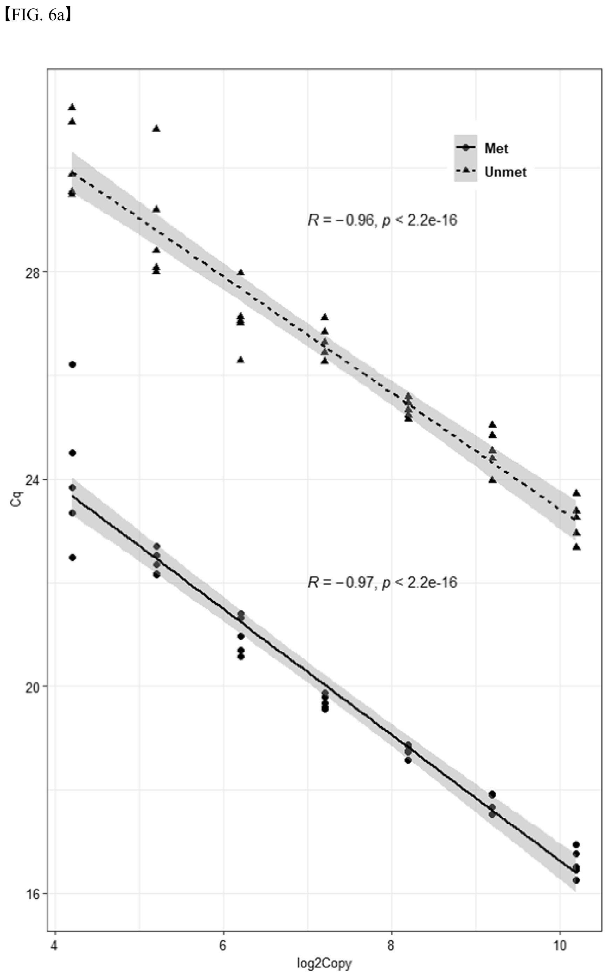

As shown in FIG. 6a, the Cq value which was reduced as each concentration increased in sulfite-converted methylated (Huh-1 gDNA) and unmethylated (Buffy Coate gDNA) gDNA could be verified, and it was verified that the correlation of PCR amplification according to the concentration was statistically significant (the Cq value was confirmed as low, since PCR amplification occurs faster as the concentration of the used specimen was higher). Theoretically, the number of the strands of 1 ng DNA was 304 strands, and the lowest amount of DNA used in the above experiment, 0.0625 ng, is 19 strands, indicating that up to 19 strands can be stably detected.

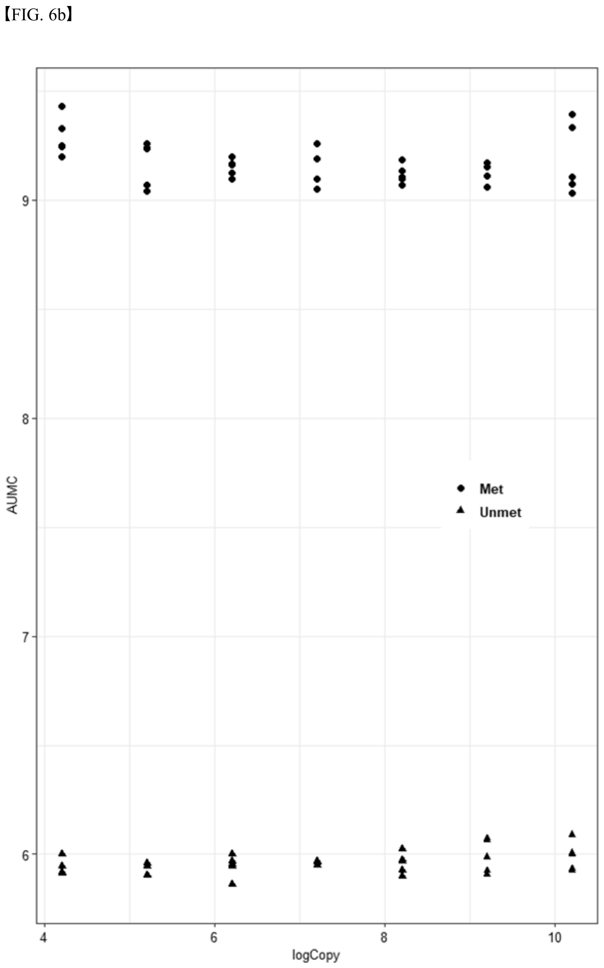

As shown in FIG. 6b, the AUMC (area under the melting curve) value based on a normalized melting point curve was calculated for the PCR amplification products of the used specimen, and it was verified that the AUMC for each methylation feature was similarly shown in all the concentration sections of methylated and unmethylated gDNA.

## (2) Analysis of minimal limit of detection (2)

Sulfite conversion was performed for Human Methylated & Unmethylated DNA Set (Cat. # D5014) products of Zymo Research company by utilizing EZ DNA Methylation-Lightning™ Kit (Cat. # D5030) of the same company, and then, quantification was performed by using Qubit™ ssDNA Assay Kit (Cat. # Q10212). After that, the sulfite-converted Methylated & Non-methylated DNA was diluted to a level of 0.1ng/uL with Nuclease-free Water (Cat. # AM9937), respectively, and then, Serial Dilution was conducted by 4 folds by the method for diluting the Methylated DNA in the Non-methylated DNA to produce specimens of Methylated DNA 100%, 25%, 6.25%, 1.56%, 0.39%, and 0%.

The exemplary sequences of the primer set produced for Marker 1 which is the biomarker according to one embodiment of the present application were shown in Table 5. In each primer sequence, sites corresponding to CpG or Non-CpG cytosine of Marker 1 were written in uppercase letters, and other bases were written in lowercase letters.

[Table 5]

| name | Seq (5'->3') | SEQ ID NO. |
|---|---|---|
| Methylated Forward Primer for Marker 1 | gagaaggTtTtggagaTTtTtgtaaTag | 10 |
| Methylated Reverse Primer for Marker 1 | ctccttAcaAaAcCGACGcCG | 11 |
| Unmethylated Reverse Primer for Marker 1 | tccttAcaAaAcCAACAcCA | 12 |

PCR Mixture used for the minimal limit of detection (LoD) experiment was prepared by using the primers of Table 5 as follows (based on 1 rxn):

Master Mix (2X): 12.5 μL (Final Conc. 1X)
LightCycler (20X): 1.25 μL (Final Conc. 1X)
Primer Mixture (the concentration of each primer is 12 μM): 1.25 μL (Final Conc. 0.6 μM)
Total: 15 μL

The prepared PCR Mixture was aliquoted by 15 μL in each well of a 96-well plate, and then the methylated DNA 100%, 25%, 6.25%, 1.56%, 0.39%, and 0% specimen at a concentration of 0.1 ng/μL was aliquoted in each well by 10 μL. PCR reaction and HRM analysis were performed under the following conditions:

95°C 5 min / 95°C 10 sec - 63°C 30 sec - 72°C 15 sec (39 Cycle): PCR reaction
72°C 5 min / 60°C - 95°C (measuring a fluorescence value, after reaction for 10 seconds by increasing by 0.2°C): HRM analysis

TS-AUMC (Temperature-shifted Area under the Melting Curve) according to the ratio of the used methylated DNA was confirmed, and the minimal limit of detection (LoD) measuring methylated DNA capable of being detected when unmethylated DNA is present in the background was verified.

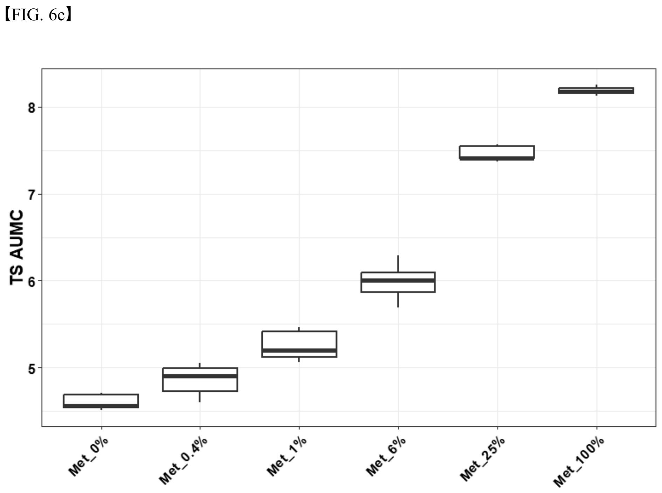

As shown in FIG. 6c, the result showed that 0% and the resolution of the Temperature-shifted AUMC value which is Methylation Signal value were verified even under the environment where 99.6% of unmethylated strands were present. Specifically, if the 0.4% level in DNA of 1ng of input DNA is converted to the number of copies, it is calculated as the level of target strand 1.2 Copy, and therefore, the method for measuring a methylation level of the biomarkers established in Example 5 showed an effect capable of detecting a trace amount of methylation strands even when a large amount of unmethylated strands were present.

### Example 7. Liver cancer diagnosis using tissue sample

gDNA extraction and sulfite conversion were performed from normal and tumor tissues collected from liver cancer (LIHC) (normal;120, tumor;115), lung cancer (LUAD) (normal;8, tumor;9), colorectal cancer (COAD) (normal;10, tumor;10), kidney cancer (KIRP and KIRC) (normal;16, tumor;15), bladder cancer (BLCA) (normal;10, tumor;9), thyroid cancer (THYM) (normal;6, tumor;6) patients. The kidney cancer was classified into kidney renal papillary cell carcinoma (KIRP) (normal;9, tumor;5) or kidney renal clear cell carcinoma (KIRC) (normal;7, tumor; 10).

Specifically, using the primers of Table 4, PCR Mixture was prepared as follows (based on 1 rxn):

Master Mix (2X): 12.5 $\mu$L (Final Conc. 1X)
EvaGreen (20X): 1.25 $\mu$L (Final Conc. 1X)
Methylated Forward Primer (10 uM): 1 $\mu$L (Final Conc. 0.4 uM)
Methylated Reverse Primer (10 uM): 1 $\mu$L (Final Conc. 0.4 uM)
Unmethylated Forward Primer (10 uM): 0.5 $\mu$L (Final Conc. 0.2 uM)
NFW (nuclease-free water): 5.75 $\mu$L
Total: 22 $\mu$L

The prepared sample was quantified and diluted to 0.33 ng/$\mu$L, and 3 $\mu$L of the corresponding sample was added. PCR reaction and HRM analysis were performed under the following conditions:

95°C 5 min / 95°C 20 sec - 69°C 40 sec (20 Cycle, starting at 69°C, decreasing by 0.3°C per Cycle, and ending at 63°C): PCR reaction

95°C 20 sec - 63°C 40 sec (40 Cycle, progressing constantly at 63°C): PCR reaction

63°C 5 min / 60°C - 95°C (measuring a fluorescence value, after reaction for 10 seconds by increasing by 0.2°C) : HRM analysis

By applying the MS-HRM analysis method established in Example 5 for each specimen, AUMC (Area under the Melting Curve) was derived from the normalized melting point curve.

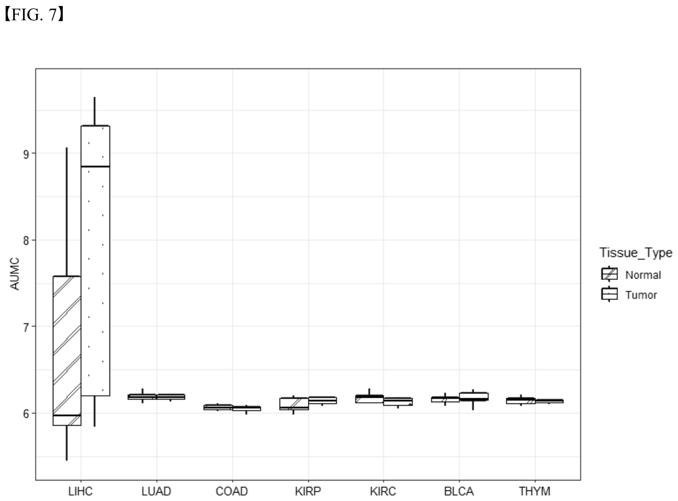

As shown in FIG. 7, the biomarker according to one embodiment of the present application showed a significantly high methylation level in the liver cancer tissue, and accordingly, it can be utilized for liver cancer diagnosis.

### Example 8. Blood sample analysis of healthy human

For sample preparation, gDNA extraction and sulfite conversion were performed from Buffy Coat collected from 55 healthy people. In order to measure the methylation level of Marker 1 according to one embodiment of the present application, using the primers of Table 4, PCR Mixture was prepared as follows (based on 1 rxn):

Master Mix (2X): 12.5 $\mu$L (Final Conc. 1X)
EvaGreen (20X): 1.25 $\mu$L (Final Conc. 1X)
Methylated Forward Primer (10 uM): 1 $\mu$L (Final Conc. 0.4 uM)
Methylated Reverse Primer (10 uM): 1 $\mu$L (Final Conc. 0.4 uM)
Unmethylated Forward Primer (10 uM): 0.5 $\mu$L (Final Conc. 0.2 uM)
NFW (nuclease-free water): 5.75 $\mu$L
Total: 22 $\mu$L

3 $\mu$L of Buffy Coat sample at a concentration of 0.33 ng/$\mu$L was added, and also 3 $\mu$L of EpiTect Unmethylated

DNA and Methylated DNA were added to each well containing PCR Mixture. PCR reaction and HRM analysis were performed under the following conditions:

95°C 5 min / 95°C 20 sec - 69°C 40 sec (20 Cycle, starting at 69°C, decreasing by 0.3°C per Cycle, and ending at 63°C): PCR reaction
95°C 20 sec - 63°C 40 sec (40 Cycle, progressing constantly at 63°C): PCR reaction
63°C 5 min / 60°C - 95°C (measuring a fluorescence value, after reaction for 10 seconds by increasing by 0.2°C) : HRM analysis

By applying the MS-HRM analysis method established in Example 5 to each sample, the melting peak was derived by differentiating the normalized melting curves with respect to temperature and taking their negative values, and then the AUMC was calculated.

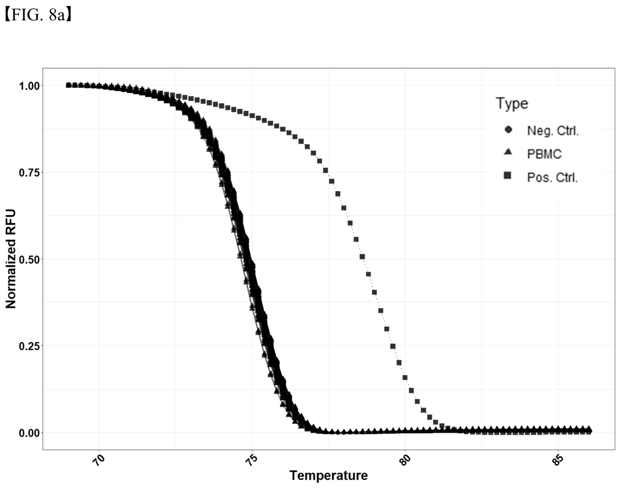

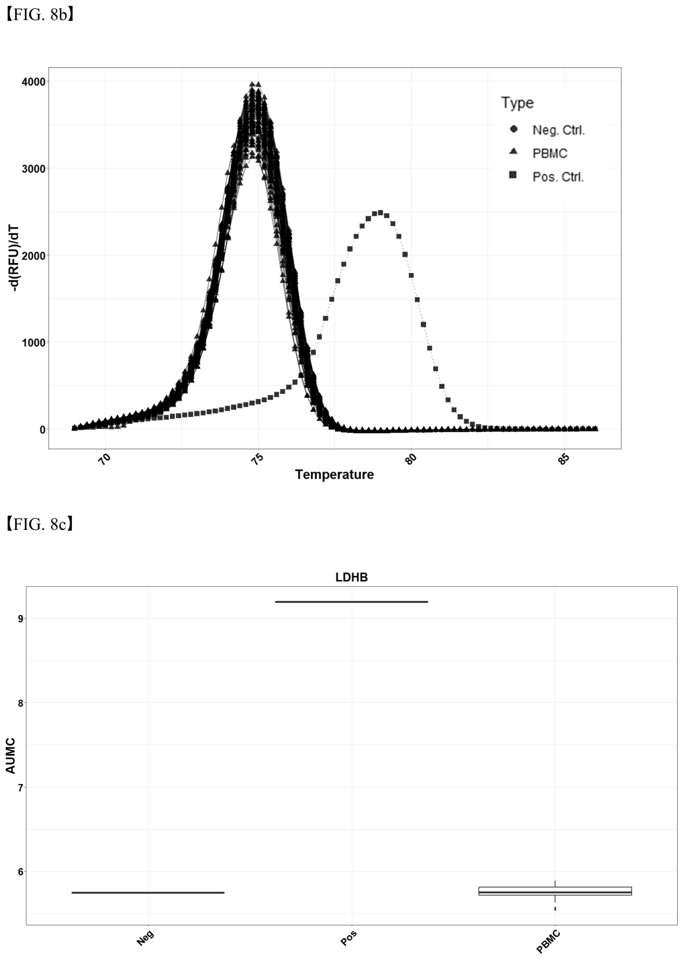

As shown in FIG. 8, all the Buffy Coat gDNA of the 55 healthy people showed the same melting point curve and melting peak results as the unmethylated DNA, and this indicates that the methylation level for the biomarker according to one embodiment of the present application was not shown in blood cells of normal people.

**Example 9. Liver cancer diagnosis using blood sample**

**(1) Analysis of accuracy of liver cancer diagnosis (1)**

Specimens for applying to the MS-HRM analysis method were prepared by performing sulfite conversion for cfDNA extracted from plasma of a total of 81 healthy people and 319 HCC patients. In order to measure the methylation level of Marker 1 according to one embodiment of the present application in each sample, PCR Mixture was prepared as follows using the primers of Table 4 (based on 1 rxn):

Master Mix (2X): 12.5 $\mu$L (Final Conc. 1X)
EvaGreen (20X): 1.25 $\mu$L (Final Conc. 1X)
Methylated Forward Primer (10 uM): 1 $\mu$L (Final Conc. 0.4 uM)
Methylated Reverse Primer (10 uM): 1 $\mu$L (Final Conc. 0.4 uM)
Unmethylated Forward Primer (10 uM): 0.5 $\mu$L (Final Conc. 0.2 uM)
NFW (nuclease-free water): 5.75 $\mu$L
Total: 22 $\mu$L

The control nucleic acid sample at 0.33 ng/$\mu$L was aliquoted in each well by 3 $\mu$L, and PCR reaction and HRM analysis were performed under the following conditions:

95°C 5 min / 95°C 20 sec - 69°C 40 sec (20 Cycle, starting at 69°C, decreasing by 0.3°C per Cycle, and ending at 63°C): PCR reaction
95°C 20 sec - 63°C 40 sec (40 Cycle, progressing constantly at 63°C): PCR reaction
63°C 5 min / 60°C - 95°C (measuring a fluorescence value, after reaction for 10 seconds by increasing by 0.2°C) : HRM analysis

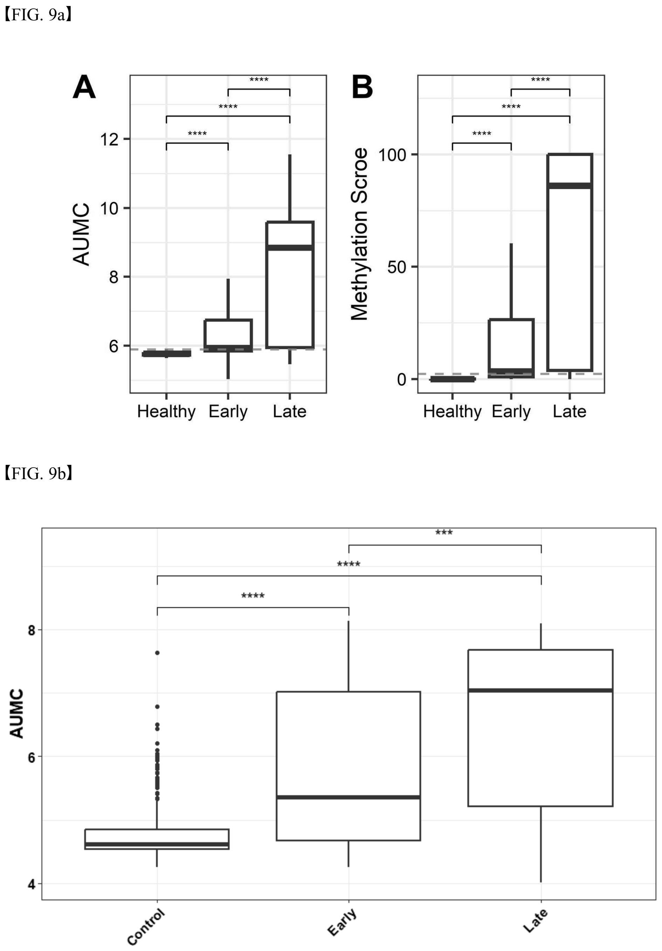

By applying the MS-HRM analysis method established in Example 5 to each specimen, AUMC was derived from the normalized melting point curve. Based on the AUMC of 81 healthy people, the cut-off was set at the 97th Percentile, and the AUMC distribution of the healthy people (Healthy) and early, middle and late liver cancer patients was shown as a boxplot in A of FIG. 9a. In addition, the methylation level was quantified based on the AUMC of the 100% methylated control sample and 100% unmethylated control sample as the following Equation 1, and shown in B of FIG. 9a. At this time, when the methylation level was less than 0, it was calculated as 0, and when the methylation level was 100 or more, it was calculated as 100. In FIG. 9a, the set cut-off value was indicated by a dotted line.

[Equation 1]

$$\text{Methylation level} = (AUMC_{sample} - AUMC_{unmet.Ctrl})/(AUMC_{Met.Ctrl} - AUMC_{Unmet.Ctrl})$$

$AUMC_{sample}$ : AUMC value of sample

$AUMC_{unmet.Ctrl}$ : AUMC value of 100% unmethylated control sample

$AUMC_{Met.Ctrl}$ : AUMC value of 100% methylated control sample

As shown in FIG. 9a, the distribution of AUMC between the healthy human group and the HCC patient group showed a statistically significant difference based on t-test, and it was verified that the methylation level of Marker 1 according to one embodiment of the present application predicted a risk of developing liver cancer accurately.

**(2) Analysis of accuracy of liver cancer diagnosis (2)**

For a total of 420 clinical specimens (healthy control group; 89 people, high risk group; 196 people, liver cancer group; 135 people), specimens were prepared by the same method as (1) of Example 9, and the AUMC analysis result using the primers of Table 5 was shown in FIG. 9b. As shown in FIG. 9b, a statistically significant increase in AUMC value (methylated level in the corresponding specimen) was confirmed in the early liver cancer group (BCLC 0-A) and late liver cancer group (BCLC B-D) compared to the control group.

In addition, based on the TS-AUMC value observed in the healthy control group from the clinical data based on the produced blood sample, the cut-off at a level of 95% specificity was set, and based on this, the sensitivity and specificity were calculated by a process of calculating sensitivity by BCLC stage of the liver cancer patients and shown in Table 6. As shown in Table 6, Marker 1 which is the biomarker according to one embodiment of the present application, had an effect of showing sensitivity of 50% or more and 70% or more, respectively, in the early and late liver cancer patients at a high specificity level, and thus, liver cancer could be diagnosed with excellent accuracy.

[Table 6]

| Type | Sensitivity | | | | | Specificity |
|---|---|---|---|---|---|---|
| Stage | BCLC 0 | BCLC A | BCLC B | BCLC C-D | Total | Healthy |
| Positive | 8 | 20 | 18 | 41 | 87 | 5 |
| Negative | 6 | 20 | 7 | 15 | 48 | 84 |
| Total | 14 | 40 | 25 | 56 | 135 | 89 |
| Se. \| Sp. | 57.1% | 50.0% | 72.0% | 73.2% | 64.4% | 94.4% |

**Example 10. Comparison with conventional liver cancer diagnosis marker**

The HCC patients were classified depending on the BCLC Stage, and were classified into Early and Late Stage (Early; BCLC 0 & A, Late; BCLC B, C & D). The diagnosis ability of the analysis method developed in the present application was compared to AFP numerical value confirmation which is a conventional liver cancer examination method, according to the HCC pathological stage of the patients. Based on the 97th Percentile of the AUMC value and AFP 20 or more confirmed in 81 healthy people, the sensitivity and specificity for HCC positive/negative determination were shown in Table 7.

[Table 7]

| Marker | | Total | Se_Early | Late | Se_Late | HCC | Se_Total | Healthy | Sp |
|---|---|---|---|---|---|---|---|---|---|
| Marker 1 | Positive | 118 | 62.4% | 101 | 77.7% | 219 | 68.7% | 3 | 96.3% |
| | Negative | 71 | | 29 | | 100 | | 78 | |
| Total | | 189 | | 130 | | 319 | | 81 | |

(continued)

| Marker | Total | Early | Se$_{Early}$ | Late | Se$_{Late}$ | HCC | Se$_{Total}$ | Healthy | Sp |
|---|---|---|---|---|---|---|---|---|---|
| AFP | Positive | 61 | 32.3% | 83 | 63.9% | 144 | 45.1% | - | - |
| | Negative | 128 | | 47 | | 175 | | - | |
| Total | | 189 | | 130 | | 319 | | 81 | |
| Combination | Positive | 137 | 72.5% | 113 | 86.9% | 250 | 78.4% | - | - |
| | Negative | 52 | | 17 | | 69 | | - | |
| Total | | 189 | | 130 | | 319 | | 81 | |

As shown in Table 7, it could be confirmed that the diagnosis sensitivity of the liver cancer examination method based on the biomarker according to one embodiment of the present application was higher by about 23.6% in the entire liver cancer patients than the AFP examination which is the conventional liver cancer examination method, and in particular, higher sensitivity by about 30.1% than the AFP examination was confirmed in the early liver cancer patients. In addition, it was confirmed that the overall diagnosis sensitivity increased exceptionally, when the liver cancer examination method using the biomarker according to one embodiment of the present application and the AFP examination method were performed together, and this can help liver cancer diagnosis by being performed together with the conventional examination method in the aspect of blood-based liquid biopsy.

**Example 11. Improvement of diagnosis performance by combination of biomarkers**

**(1) Liver cancer diagnosis by combination of Marker 1 and Marker 2**

The HCC patients were classified depending on the BCLC Stage, and classified into Early and Late Stage (Early; BCLC 0 & A, Late; BCLC B, C & D). The liver cancer diagnosis ability when Marker 1 and Marker 2 according to one embodiment of the present application were combined was compared according to the HCC pathological stage of the patients and shown in Table 8.

[Table 8]

| Marker | Total | Early | Se$_{Early}$ | Late | Se$_{Late}$ | HCC | Se$_{Total}$ | Healthy | Sp |
|---|---|---|---|---|---|---|---|---|---|
| Marker 1 | Positive | 121 | 63.0% | 103 | 77.4% | 224 | 68.9% | 3 | 96.3% |
| | Negative | 71 | | 30 | | 101 | | 79 | |
| Total | | 192 | | 133 | | 325 | | 82 | |
| Marker 2 | Positive | 71 | 37.0% | 90 | 67.7% | 161 | 49.5% | 1 | 98.8% |
| | Negative | 121 | | 43 | | 164 | | 81 | |
| Total | | 192 | | 133 | | 325 | | 82 | |
| Combination | Positive | 143 | 74.5% | 115 | 86.5% | 258 | 79.4% | 4 | 95.1% |
| | Negative | 49 | | 18 | | 67 | | 78 | |
| Total | | 192 | | 133 | | 325 | | 82 | |

As shown in Table 8, when Marker 1 according to one embodiment of the present application was used, superior sensitivity was confirmed in all the liver cancer pathological stages compared to Marker 2, and additionally, when the two markers were combined and used, the specificity was maintained equally, and the sensitivity increased by 10% or more, and thus, it showed that improvement of the liver cancer diagnosis performance could be promoted.

**(2) Liver cancer diagnosis by combination of Marker 1 and various biomarkers**

Whether the liver cancer diagnosis accuracy was improved or not was verified by combining Marker 1 which is the biomarker according to one embodiment of the present application, with various biomarkers such as AFP, age and gender and the like. As a control group, the conventional MS-HRM method was used. Specifically, among the produced data, a logistic regression analysis using the glm() function was performed in R based on specimens in which all of the age, gender and AFP examination numerical values were all present, and a diagnosis prediction model was constructed by combining it with the TS-AUMC value and the roc() function was utilized to confirm the clinical performance.

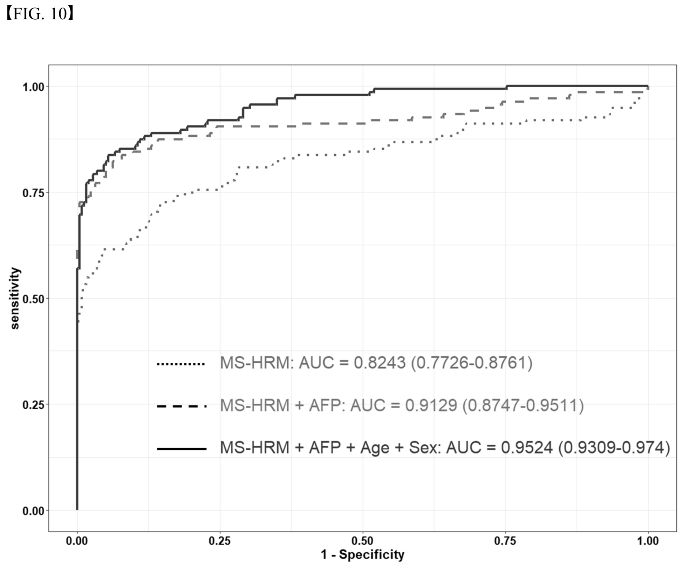

As shown in FIG. 10, Marker 1 which is the biomarker according to one embodiment of the present application showed excellent liver cancer diagnosis ability even when used alone, and when the liver cancer group was predicted based on the logistic regression model by combining it with the AFP examination method, age information and gender information, a synergistic effect in which the maximal AUC increased to 0.9524 (0.9309-0.9740) was shown.

## Example 12. Liver cancer specificity of target methylation site

In order to confirm the liver cancer specificity of target site of Marker 1 according to one embodiment of the present application, the methylation data of the TCGA and GEO databases used in Example 4 were used to analyze the mean methylation level by cancer type.

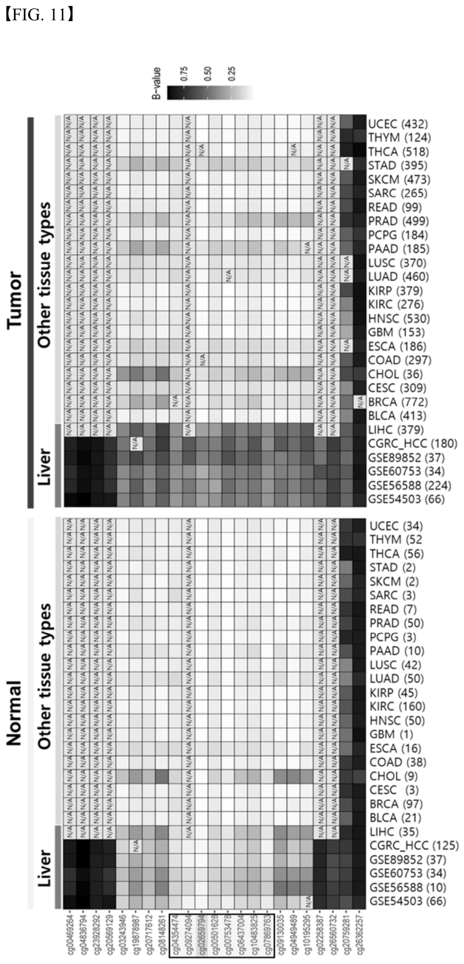

Specifically, the result of confirming the methylation level by each data set with a heatmap using ggplot2 R package in R software was shown in FIG. 11 (X-axis: sample, Y-axis: Probe ID in LDHB gene). In FIG. 11, the X-axis refers to the normal and cancer tissue samples of 23 cancer type data sets including liver cancer, and the Y-axis refers to the biomarker, and the color of the matrix refers to the methylation level, and the boxed part in the area where the Probe ID was indicated represents CpG sites positioned in the 21810279th to 21810280th, 21810450th to 21810451st, 21810458th to 21810459th, 21810489th to 21810490th, 21810600th to 21810601st, 21810750th to 21810751st, 21810759th to 21810760th, and 21810791st to 21810792nd sequences of the human chromosome 12 among the CGI area comprised in Marker 1 according to one embodiment of the present application, and the shaded part represents the target site of the 21810750th to 21810751st CGI.

As shown in FIG. 11, it was highly methylated only in the liver cancer sample in the CGI (CpG island) area comprised in the 21810279th to 21810792nd sequences of the chromosome 12, and a low methylated pattern was shown in the normal liver and other cancer types, and thus, high liver cancer specificity can be secured through the methylation level of the target site. On the other hand, other areas did not show a liver cancer-specific methylation state, as they were highly methylated in most of specimens such as cg26362257 (21788425th to 21788426th sequences of the chromosome 12), etc., or they were highly methylated in pancreatic adenocarcinoma (PAAD) including cholangiocarcinoma (CHOL) and prostate adenocarcinoma (PRAD) such as cg03243946 (21811034th to 21811035th sequences of the chromosome 12) and cg10195295 (21809973th to 21809974th sequences of the chromosome 12), etc., and the like.

Therefore, as a result of verifying the mean methylation value in each cancer type, it was verified that only in the Marker 1 region according to one embodiment of the present application had liver cancer specificity.

## Example 13. Verification of liver cancer diagnostic ability of biomarkers

Specimens for applying to the MS-HRM analysis method by sulfite converting cfDNA extracted from plasma of a total of 210 healthy people, 176 high-risk patients and 133 liver cancer patients were prepared. The exemplary sequences of the primer set produced for Marker 2 were shown in Table 9, and the exemplary sequences of the primer set produced for Marker 3 were shown in Table 10. In each primer sequence, sites corresponding to CpG or Non-CpG cytosine of Marker 3 were written in uppercase letters, and other bases were written in lowercase letters.

[Table 9]

| name | Seq (5'->3') | SEQ ID NO. |
|---|---|---|
| Methylated Forward Primer for Marker 2 | TTatggCGggTTtgggTTtgggTtT | 13 |
| Methylated Reverse Primer for Marker 2 | caAcccctAAcaAatAatAcaAcC | 14 |

[Table 10]

| name | Seq (5'->3') | SEQ ID NO. |
|---|---|---|
| Methylated Forward Primer for Marker 3 | GgatTagggagtggTttgtaaT | 15 |
| Methylated Reverse Primer for Marker 3 | ccCGctctcttcttCGtA | 16 |
| Unmethylated Reverse Primer for Marker 3 | acccCActctcttcttCAtA | 17 |

In order to measure the methylation level of Marker 2 according to one embodiment of the present application in each sample, PCR Mixture was prepared as Example 5 using the primers of Table 7, and PCR reaction and HRM analysis were performed under the same conditions.

In addition, in order to measure the methylation level of Marker 3 according to one embodiment of the present application, PCR Mixture was prepared as follows using the primers of Table 8 (based on 1 rxn):

Master Mix (2X): 12.5 µL (Final Conc. 1X)
EvaGreen (20X): 1.25 µL (Final Conc. 1X)
Methylated Forward Primer (10 uM): 1 µL (Final Conc. 0.4 uM)
Methylated Reverse Primer (10 uM): 1 µL (Final Conc. 0.4 uM)
Unmethylated Reverse Primer (10 uM): 0.5 µL (Final Conc. 0.2 uM)
NFW (nuclease-free water): 5.75 µL
Total: 22 µL

A control nucleic acid sample of 0.33 ng/µL was aliquoted in each well by 3 µL, and PCR reaction and HRM analysis were performed under the following conditions:

95°C 5 min / 95°C 20 sec - 61°C 30 sec - 72°C 30 sec (50 Cycle): PCR reaction
72°C 5 min / 60°C - 95°C (measuring a fluorescence value, after reaction for 10 seconds by increasing by 0.2°C): HRM analysis

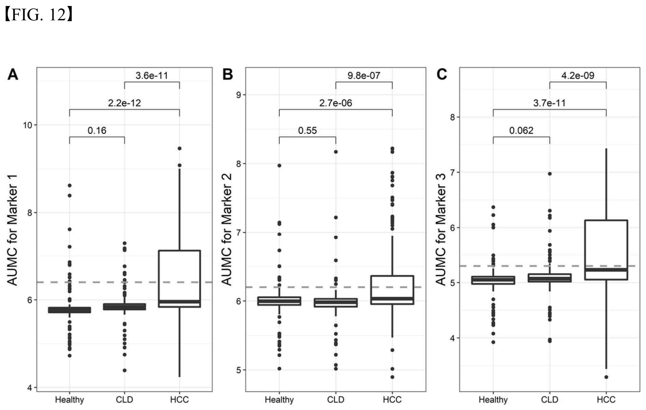

By applying the MS-HRM analysis method established in Example 5 into each specimen, AUMC was derived from the normalized melting point curve. The cut-off was set at the 95th Percentile based on the AUMC of 210 healthy people, and the AUMC distribution in the healthy people, high-risk patients and liver cancer patients was shown in FIG. 12 as a boxplot. In FIG. 12, the set cut-off value was indicated by a dotted line.

As shown in Fig. 12, as high AUMC distribution was shown in the liver cancer patient group statistically significantly based on the t-test between the health human group, high-risk patient and liver cancer patient groups, it was verified that the methylation levels of Marker 2 and Marker 3 according to one embodiment of the present application predicted a risk of developing liver cancer accurately.

**Claims**

1. A composition for predicting a risk of developing liver cancer, comprising an agent capable of measuring a methylation level of at least one CpG site comprised in the 21810279th to 21810792nd sequence region of the human chromosome 12 in a biological sample.

2. The composition according to claim 1, wherein the CpG site comprises a CpG site positioned in the 21810750th to 21810751st sequences of the human chromosome 12.

3. The composition according to claim 1, wherein the region has the sequence represented by SEQ ID NO: 1.

4. The composition according to claim 1, further comprising an agent capable of measuring a methylation level of at least one CpG site comprised in the 29298021st to 29298631st sequence region of the human chromosome 17 in the biological sample; and/or an agent capable of measuring a methylation level of at least one CpG site comprised in the 19738547th to 19739846th sequence region of the human chromosome 19 in the biological sample.

5. The composition according to claim 4, wherein at least one CpG site comprised in the 29298021st to 29298631st sequence region of the human chromosome 17 comprises a CpG site positioned in the 29298115th to 29298116th, 29298118th to 29298119th, 29298124th to 29298125th, 29298136th to 29298137th, 29298142nd to 29298143rd, or 29298184th to 29298185th sequences of the human chromosome 17, and
wherein at least one CpG site comprised in the 19738547th to 19739846th sequences of the human chromosome 19 comprises a CpG site positioned in the 19739407th to 19739408th sequences of the human chromosome 19.

6. The composition according to claim 4, wherein the 29298021st to 29298631st sequence region of the human chromosome 17 has the sequence represented by SEQ ID NO: 2, and the 19738547th to 19739846th sequence region of the human chromosome 19 has the sequence represented by SEQ ID NO: 3.

7. The composition according to claim 1, further comprising an agent capable of measuring the concentration of alpha fetoprotein (AFP) in the biological sample.

8. The composition according to claim 1, wherein the agent is a primer for amplifying a target methylation site of the sequence region.

9. The composition according to claim 8, wherein the primer comprises a forward primer and a revers primer,

   wherein one of the forward primer and the reverse primer comprises a methylation primer and an unmethylation primer,
   wherein the methylation primer comprises at least one CpG recognition site which recognizes a CpG sequence comprised in a sequence with a predetermined length comprising the target methylation site,
   wherein the unmethylation primer comprises at least one TpG recognition site which recognizes a TpG sequence comprised in a sequence with a predetermined length comprising the target methylation site, and
   wherein the TpG sequence of the sequence with the predetermined length comprising the target methylation site is converted from the unmethylated CpG sequence of the sequence with the predetermined length comprising the target methylation site.

10. The composition according to claim 9, wherein the TpG sequence is converted by a modifying agent that modifies a methylated CpG sequence and an unmethylated CpG sequence differently from each other.

11. The composition according to claim 10, wherein the modifying agent converts an unmethylated cytosine residue into thymine.

12. The composition according to claim 1, wherein the biological sample is one or more selected from the group consisting of blood, plasma, serum, platelets, tissue, cells, feces and urine.

13. The composition according to claim 1, wherein the sequence region is comprised in cell free DNA (cfDNA).

14. The composition according to claim 1, wherein the liver cancer is hepatocellular carcinoma (HCC).

15. A kit for predicting a risk of developing liver cancer, comprising the composition according to any one of claims 1 to 14.

16. A method for providing information for predicting a risk of developing liver cancer, comprising a step of measuring a methylation level of at least one CpG site comprised in the 21810279th to 21810792nd sequence region of the human chromosome 12 in a biological sample isolated from a subject.

17. The method according to claim 16, wherein the CpG site comprises a CpG site positioned in the 21810750th to 21810751st sequences of the human chromosome 12.

18. The method according to claim 16, wherein the region has the sequence represented by SEQ ID NO: 1.

19. The method according to claim 16, further comprising a step of measuring a methylation level of at least one CpG site comprised in the 29298021st to 29298631st sequence regions of the human chromosome 17 in the biological sample; and/or a step of measuring a methylation level of at least one CpG site comprised in the 19738547th to 19739846th sequence region of the human chromosome 19 in the biological sample.

20. The method according to claim 19, wherein at least one CpG site comprised in the 29298021st to 29298631st sequence region of the human chromosome 17 comprises a CpG site positioned in the 29298115th to 29298116th, 29298118th to 29298119th, 29298124th to 29298125th, 29298136th to 29298137th, 29298142nd to 29298143rd, or 29298184th to 29298185th sequences of the human chromosome 17, and wherein at least one CpG site comprised in the 19738547th to 19739846th sequences of the human chromosome 19 comprises a CpG site positioned in the 19739407th to 19739408th sequences of the human chromosome 19.

21. The method according to claim 19, wherein the 29298021st to 29298631st sequence region of the human chromosome 17 has the sequence represented by SEQ ID NO: 2, and the 19738547th to 19739846th sequence region of the human chromosome 19 has the sequence represented by SEQ ID NO: 3.

22. The method according to claim 16, further comprising a step of measuring the concentration of alpha fetoprotein (AFP) in the biological sample, a step of collecting gender information of the subject, and/or a step of collecting age information of the subject.

23. The method according to claim 16, wherein the methylation level is measured by using a primer for amplifying a target methylation site of the sequence.

24. The method according to claim 23, wherein the primer comprises a forward primer and a revers primer,

wherein one of the forward primer and the reverse primer comprises a methylation primer and an unmethylation primer,
wherein the methylation primer comprises at least one CpG recognition site which recognizes a CpG sequence comprised in a sequence with a predetermined length comprising the target methylation site,
wherein the unmethylation primer comprises at least one TpG recognition site which recognizes a TpG sequence comprised in a sequence with a predetermined length comprising the target methylation site, and
wherein the TpG sequence of the sequence with the predetermined length comprising the target methylation site is converted from the unmethylated CpG sequence comprised in the sequence with the predetermined length comprising the target methylation site.

25. The method according to claim 16, further comprising a step of quantifying the methylation level of the CpG site.

26. The method according to claim 25, wherein the step of quantifying quantifies the methylation level of the CpG site by comparing AUCs (Area under the curves) of normalized melting curves of the biological sample; a sample in which the CpG site is 100% methylated; and a sample in which the CpG site is 100% unmethylated.

27. The method according to claim 16, further comprising a step of obtaining a melt curve of the biological sample;

a step of obtaining a normalized melt curve of a biological sample in which the content ratio of the sequence in which the CpG site is methylated and the sequence in which the CpG site is unmethylated is known; and
a step of quantifying the methylation level of the CpG site by comparing the melt curve of the biological sample and the normalized melt curve.

28. The method according to claim 16, further comprising a step of comparing the methylation level with the methylation level of a control group.

29. The method according to claim 16, further comprising a step of comparing the methylation level with the methylation level of a normal control group, wherein the method provides information that the subject has a risk of developing liver cancer, when the methylation level is higher than the methylation level of the normal control group.

30. The method according to claim 16, wherein the biological sample is one or more selected from the group consisting of blood, plasma, serum, platelets, tissue, cells, feces and urine.

31. The method according to claim 16, wherein the sequence is comprised in cell free DNA (cfDNA).

32. The method according to claim 16, wherein the liver cancer is hepatocellular carcinoma (HCC).

【FIG. 1】

marker 1

【FIG. 2】

【FIG. 3a】

【FIG. 3b】

**Type**
☐ Normal
■ Tumor

**Dataset**
▨ GSE54503    ■ GSE89852
▨ GSE56588    ▨ CGRC HCC
▨ GSE60753    ☐ TCGA HCC

【FIG. 3c】

**Type**
☐ Normal
■ Tumor

**Dataset**
▨ GSE54503    ■ GSE89852
▨ GSE56588    ▨ CGRC HCC
▨ GSE60753    ☐ TCGA HCC

【FIG. 4】

【FIG. 5a】

【FIG. 5b】

【FIG. 5c】

【FIG. 6a】

【FIG. 6b】

【FIG. 6c】

【FIG. 7】

【FIG. 8a】

【FIG. 8b】

【FIG. 8c】

【FIG. 9a】

【FIG. 9b】

【FIG. 10】

【FIG. 11】

【FIG. 12】

## INTERNATIONAL SEARCH REPORT

| International application No. |
| --- |
| **PCT/KR2024/001115** |

### A. CLASSIFICATION OF SUBJECT MATTER

**C12Q 1/6886**(2018.01)i

According to International Patent Classification (IPC) or to both national classification and IPC

### B. FIELDS SEARCHED

Minimum documentation searched (classification system followed by classification symbols)

C12Q 1/6886(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: DNA 메틸화(DNA methylation), 액체 생검(liquid biopsy), 간암(liver cancer), 간세포암(hepatocellular carcinoma, HCC), 바이오마커(biomarker), 세포 유리 DNA(cell-free DNA, cfDNA), CpG 사이트(CpG site), 프로모터(promoter), 알파태아단백질(Alpha Fetoprotein, AFP), LDHB, RNF135

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2021-0044159 A (GENCURIX INC.) 22 April 2021 (2021-04-22) See claims 1-5, 7 and 9; paragraphs [0005], [0008], [0028], [0032], [0040], [0043], [0051], [0055], [0056], [0081], [0084] and [0085]; and table 1. | 1,3,7-16,18,22-32 |
| Y | | 4,6,19,21 |
| A | | 2,5,17,20 |
| Y | WANG, X. et al. RNF135 promoter methylation is associated with immune infiltration and prognosis in hepatocellular carcinoma. Frontiers in Oncology. 25 January 2022 (publication date), vol. 11, article no. 752511, inner pp. 1-12. See abstract; inner pages 1-11; and Supplementary Figure 1. | 4,6,19,21 |
| A | REVILL, K. et al. Genome-wide methylation analysis and epigenetic unmasking identify tumor suppressor genes in hepatocellular carcinoma. Gastroenterology. 2013, vol. 145, no. 6, pp. 1424-1435. See pages 1427-1435. | 1-32 |

☑ Further documents are listed in the continuation of Box C.  ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **02 May 2024** | **02 May 2024** |

| Name and mailing address of the ISA/KR | Authorized officer |
| --- | --- |
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/001115**

## C.     DOCUMENTS CONSIDERED TO BE RELEVANT

| Category* | Citation of document, with indication, where appropriate, of the relevant passages | Relevant to claim No. |
|---|---|---|
| A | KR 10-2019-0059215 A (UIF (UNIVERSITY INDUSTRY FOUNDATION), YONSEI UNIVERSITY et al.) 30 May 2019 (2019-05-30)<br>    See abstract; and claims 1-9. | 1-32 |
| A | WO 2022-243566 A1 (OPHIOMICS - INVESTIGAÇÃO E DESENVOLVIMENTO EM BIOTECNOLOGIA) 24 November 2022 (2022-11-24)<br>    See abstract; and claims 1-14. | 1-32 |
| PX | KIM, S. C. et al. A circulating cell-free DNA methylation signature for the detection of hepatocellular carcinoma. Molecular Cancer. 06 October 2023 (publication date), vol. 22, no. 164, inner pp. 1-6.<br>    See abstract; and inner pages 1-5. | 1-32 |

Form PCT/ISA/210 (second sheet) (July 2022)

| **INTERNATIONAL SEARCH REPORT** | International application No. |
| --- | --- |
| | **PCT/KR2024/001115** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/KR2024/001115**

| Patent document cited in search report | | | Publication date (day/month/year) | Patent family member(s) | | | Publication date (day/month/year) |
|---|---|---|---|---|---|---|---|
| KR | 10-2021-0044159 | A | 22 April 2021 | EP | 4047102 | A2 | 24 August 2022 |
| | | | | JP | 2022-552400 | A | 15 December 2022 |
| | | | | KR | 10-2472253 | B1 | 30 November 2022 |
| | | | | WO | 2021-075797 | A2 | 22 April 2021 |
| | | | | WO | 2021-075797 | A3 | 10 June 2021 |
| KR | 10-2019-0059215 | A | 30 May 2019 | EP | 3715474 | A2 | 30 September 2020 |
| | | | | EP | 3715474 | A4 | 09 February 2022 |
| | | | | EP | 4180540 | A1 | 17 May 2023 |
| | | | | JP | 2021-503956 | A | 15 February 2021 |
| | | | | JP | 2022-164712 | A | 27 October 2022 |
| | | | | JP | 7306723 | B2 | 11 July 2023 |
| | | | | KR | 10-2019-0087344 | A | 24 July 2019 |
| | | | | KR | 10-2019-0088924 | A | 29 July 2019 |
| | | | | KR | 10-2052089 | B1 | 05 December 2019 |
| | | | | KR | 10-2103886 | B1 | 29 May 2020 |
| | | | | KR | 10-2103887 | B1 | 29 May 2020 |
| | | | | SG | 11-2020-04795 | RA | 29 June 2020 |
| | | | | US | 2021-0147943 | A1 | 20 May 2021 |
| | | | | US | 2022-0259676 | A1 | 18 August 2022 |
| | | | | WO | 2019-103421 | A2 | 31 May 2019 |
| | | | | WO | 2019-103421 | A3 | 18 July 2019 |
| WO | 2022-243566 | A1 | 24 November 2022 | CN | 117355616 | A | 05 January 2024 |

Form PCT/ISA/210 (patent family annex) (July 2022)